(19) Europäisches Patentamt
European Patent Office
Office européen des brevets

(11) **EP 2 628 018 B1**

(12) **FASCICULE DE BREVET EUROPEEN**

(45) Date de publication et mention
de la délivrance du brevet:
**16.05.2018 Bulletin 2018/20**

(21) Numéro de dépôt: **11832100.9**

(22) Date de dépôt: **11.10.2011**

(51) Int Cl.:
*G01R 33/563* (2006.01)      *G01R 33/56* (2006.01)
*A61B 5/00* (2006.01)      *A61B 5/02* (2006.01)
*A61B 5/026* (2006.01)      *A61B 5/055* (2006.01)
*A61B 6/00* (2006.01)      *A61B 6/03* (2006.01)
*G01N 24/08* (2006.01)      *G06F 17/18* (2006.01)
*G06N 7/00* (2006.01)

(86) Numéro de dépôt international:
**PCT/FR2011/052374**

(87) Numéro de publication internationale:
**WO 2012/049421 (19.04.2012 Gazette 2012/16)**

(54) **SYSTÈME ET PROCÉDÉ POUR ESTIMER UNE QUANTITÉ D'INTÉRÊT D'UN SYSTÈME DYNAMIQUE ARTÈRE/TISSU/VEINE**

SYSTEM UND VERFAHREN ZUM SCHÄTZEN EINER RELEVANTEN MENGE FÜR EIN DYNAMISCHES ARTERIEN-/GEWEBE-/VENENSYSTEM

SYSTEM AND PROCESS FOR ESTIMATING A QUANTITY OF INTEREST OF A DYNAMIC ARTERY/TISSUE/VEIN SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.10.2010 FR 1058251**

(43) Date de publication de la demande:
**21.08.2013 Bulletin 2013/34**

(73) Titulaire: **Olea Medical**
**13600 La Ciotat (FR)**

(72) Inventeur: **PAUTOT, Fabrice**
**13600 La Ciotat (FR)**

(74) Mandataire: **Brun, Philippe Alexandre Georges**
**MED'iNVENT CONSULTING**
**Espace Mistral - Bât.A**
**297 avenue du Mistral**
**ZI ATHELIA IV**
**13705 La Ciotat Cedex (FR)**

(56) Documents cités:
- SCHMID V J ET AL: "Quantitative Analysis of Dynamic Contrast-Enhanced MR Images Based on Bayesian P-Splines", IEEE TRANSACTIONS ON MEDICAL IMAGING, IEEE SERVICE CENTER, PISCATAWAY, NJ, US, vol. 28, no. 6, 1 juin 2009 (2009-06-01), pages 789-798, XP011248726, ISSN: 0278-0062
- ERWAN GRAVIER ET AL: "Fully 4D motion-compensated reconstruction of cardiac SPECT imagesThis work was supported in part by the National Institutes of Health under grant no HL65425.; Fully 4D motion-compensated reconstruction of cardiac SPECT images", PHYSICS IN MEDICINE AND BIOLOGY, TAYLOR AND FRANCIS LTD. LONDON, GB, vol. 51, no. 18, 21 septembre 2006 (2006-09-21), pages 4603-4619, XP020095936, ISSN: 0031-9155, DOI: DOI:10.1088/0031-9155/51/18/010
- GREEN P J: "BAYESIAN RECONSTRUCTIONS FROM EMISSION TOMOGRAPHY DATA USING A MODIFIED EM ALGORITHM", IEEE TRANSACTIONS ON MEDICAL IMAGING, IEEE SERVICE CENTER, PISCATAWAY, NJ, US, vol. 9, no. 1, 1 mars 1990 (1990-03-01), pages 84-93, XP000116144, ISSN: 0278-0062, DOI: DOI:10.1109/42.52985

- ZANDERIGO F ET AL: "Nonlinear Stochastic Regularization to Characterize Tissue Residue Function in Bolus-Tracking MRI: Assessment and Comparison With SVD, Block-Circulant SVD, and Tikhonov", IEEE TRANSACTIONS ON BIOMEDICAL ENGINEERING, IEEE SERVICE CENTER, PISCATAWAY, NJ, USA, vol. 56, no. 5, 1 mai 2009 (2009-05-01), pages 1287-1297, XP011293074, ISSN: 0018-9294
- BENAVOLI A ET AL: "Hard-constrained versus soft-constrained parameter estimation", IEEE TRANSACTIONS ON AEROSPACE AND ELECTRONIC SYSTEMS, IEEE SERVICE CENTER, PISCATAWAY, NJ, US, vol. 42, no. 4, 1 octobre 2006 (2006-10-01), pages 1224-1239, XP011168590, ISSN: 0018-9251, DOI: 10.1109/TAES.2006.314569
- SATO M-A ET AL: "Hierarchical Bayesian estimation for MEG inverse problem", NEUROIMAGE, ACADEMIC PRESS, ORLANDO, FL, US, vol. 23, no. 3, 1 novembre 2004 (2004-11-01), pages 806-826, XP004620754, ISSN: 1053-8119, DOI: 10.1016/J.NEUROIMAGE.2004.06.037
- BENALI H ET AL: "Estimation of the Hemodynamic Response in Event-Related Functional MRI: Bayesian Networks as a Framework for Efficient Bayesian Modeling and Inference", IEEE TRANSACTIONS ON MEDICAL IMAGING, IEEE SERVICE CENTER, PISCATAWAY, NJ, US, vol. 23, no. 8, 1 août 2004 (2004-08-01), pages 959-967, XP011115625, ISSN: 0278-0062, DOI: 10.1109/TMI.2004.831221
- WOOLRICH M W ET AL: "Bayesian analysis of neuroimaging data in FSL", NEUROIMAGE, ACADEMIC PRESS, ORLANDO, FL, US, vol. 45, no. 1, 1 mars 2009 (2009-03-01), pages S173-S186, XP025947910, ISSN: 1053-8119, DOI: DOI:10.1016/J.NEUROIMAGE.2008.10.055 [extrait le 2008-11-13]
- SOURBRON ET AL: "Technical aspects of MR perfusion", EUROPEAN JOURNAL OF RADIOLOGY, ELSEVIER SCIENCE, NL, vol. 76, no. 3, 3 avril 2010 (2010-04-03), pages 304-313, XP027544933, ISSN: 0720-048X [extrait le 2010-12-01]
- NEYRAN B ET AL: "Mapping myocardial perfusion with an intravascular MR contrast agent, a robust estimation by a spatially constrained approach", COMPUTERS IN CARDIOLOGY, 2005 LYON, FRANCE SEPT. 25-28, 2005, PISCATAWAY, IEEE, NJ, USA, 25 septembre 2005 (2005-09-25), pages 407-410, XP010889858, DOI: DOI:10.1109/CIC.2005.1588123 ISBN: 978-0-7803-9337-0
- BRUNECKER ET AL: "Correcting saturation effects of the arterial input function in dynamic susceptibility contrast-enhanced MRI - a Monte Carlo simulation", MAGNETIC RESONANCE IMAGING, ELSEVIER SCIENCE, TARRYTOWN, NY, US, vol. 25, no. 9, 25 octobre 2007 (2007-10-25), pages 1300-1311, XP022314281, ISSN: 0730-725X, DOI: DOI:10.1016/J.MRI.2007.03.011
- WOOLRICH M W ET AL: "Bayesian inference of hemodynamic changes in functional arterial spin labeling data", MAGNETIC RESONANCE IN MEDICINE, ACADEMIC PRESS, DULUTH, MN, US, vol. 56, no. 4, 1 octobre 2006 (2006-10-01), pages 891-906, XP007918174, ISSN: 0740-3194, DOI: DOI:10.1002/MRM.21039 [extrait le 2006-09-08]

**Description**

L'invention concerne un système et un procédé pour estimer des paramètres hémodynamiques par application de méthodes probabilistes douces à l'imagerie de perfusion. Un tel procédé permet en outre d'estimer des fonctions de répartition complémentaire ou d'entrée artérielles et donc plus généralement toute quantité d'intérêt. L'invention se distingue notamment des procédés connus en ce quelle requière l'introduction d'informations a priori douces de nature physiologique ou hémodynamique sans contraindre ni forcer l'estimation recherchée par des hypothèses arbitraires et indésirables.

L'invention s'appuie notamment sur des techniques d'imagerie de Perfusion par Résonance Magnétique (*Perfusion Weighted Magnetic Resonance Imaging* - PW-MRI - selon une terminologie anglo-saxonne) ou par Tomodensitométrie (*Computed Tomography* - CT - selon une terminologie anglo-saxonne). Ces techniques permettent d'obtenir rapidement des informations précieuses sur l'hémodynamique d'organes tels que le cerveau ou le coeur. Ces informations sont particulièrement cruciales pour un praticien cherchant à établir un diagnostic et à prendre une décision thérapeutique dans le traitement en urgence de pathologies telles que les accidents vasculaires cérébraux.

Pour mettre en oeuvre de telles techniques, on utilise un appareil d'imagerie par Résonance Magnétique Nucléaire ou par Tomodensitométrie. Celui-ci délivre une pluralité de séquences d'images numériques d'une partie du corps, notamment du cerveau. Ledit appareil applique pour cela une combinaison d'ondes électromagnétiques à haute fréquence sur la partie du corps considérée et mesure le signal réémis par certains atomes. L'appareil permet ainsi de déterminer la composition chimique et donc la nature des tissus biologiques en chaque point (ou voxel) du volume imagé.

Des séquences d'images sont analysées au moyen d'une unité de traitement dédiée. Cette unité de traitement délivre in fine à un praticien, une estimation des paramètres hémodynamiques à partir des images de perfusion, au moyen d'une interface homme-machine adaptée. Le praticien peut ainsi réaliser un diagnostic et décider de l'action thérapeutique qu'il jugera adéquate.

Des images de perfusion par Résonance Magnétique Nucléaire ou par Tomodensitométrie sont obtenues en injectant un agent de contraste (par exemple un sel de gadolinium pour l'Imagerie par Résonance Magnétique) par voie intraveineuse et en enregistrant son bol au cours du temps au niveau de chaque voxel de l'image. Par souci de concision, nous omettrons les indices $x,y,z$ pour identifier des voxels. Par exemple, au lieu de noter $S_{x,y,z}(t)$ le signal pour un voxel de coordonnées $x,y,z$, nous le noterons simplement $S(t)$. Il est entendu que les opérations et les calculs décrits dans la suite sont généralement effectués pour chaque voxel d'intérêt, de sorte à obtenir au final des images ou des cartes représentatives des paramètres hémodynamiques que l'on cherche à estimer.

Un modèle standard permet de relier l'intensité des signaux $S(t)$ mesurée au cours du temps $t$ à la concentration $C(t)$ dudit agent de contraste.

Par exemple, en Tomodensitométrie de Perfusion, le signal pour chaque voxel est directement proportionnel à la concentration : $S(t)=k \cdot C(t)+S_0$. En Imagerie de Perfusion par Résonance Magnétique Nucléaire, il existe une relation exponentielle $S(t)=S_0 \cdot e^{-k \cdot TE \cdot C(t)}$. Dans les deux cas, $S_0$ représente l'intensité moyenne du signal avant l'arrivée de l'agent de contraste. En ce qui concerne l'imagerie par Résonance magnétique nucléaire, $k$ est une constante dépendant de la relation entre la susceptibilité paramagnétique et la concentration de l'agent de contraste dans le tissu et $TE$ est le temps d'écho (*echo time* selon une terminologie anglo-saxonne). La valeur de la constante $k$ pour chaque voxel étant inconnue, celle-ci est fixée à une valeur arbitraire pour tous les voxels d'intérêt. On obtient ainsi des estimations relatives et non pas absolues. Ces informations relatives restent toutefois pertinentes puisque l'on est intéressé principalement par la variation relative de ces valeurs dans l'espace, en particulier entre les tissus sains et les tissus pathologiques.

De manière générale, nous noterons $S(t)=\Psi(C(t),\Theta_S)$ le modèle reliant le signal théorique $S(t)$ à la concentration de l'agent de contraste théorique $C(t)$, $\Theta_S$ étant le vecteur des paramètres libres de ce modèle. Par exemple, dans les cas de l'imagerie de perfusion par résonance magnétique et de l'imagerie de perfusion par tomodensitométrie, $\Theta_S=(S_0)$.

La conservation de la masse de l'agent de contraste dans le volume de tissu contenu dans chaque voxel à

$$\frac{dC(t)}{dt} = BF . \left[ C_a(t) - C_v(t) \right] . \quad C_a(t)$$

chaque instant s'écrit est la concentration de l'agent de contraste dans l'artère alimentant le volume de tissu (fonction d'entrée artérielle ou *Arterial Input Fonction* - AIF - selon une terminologie anglo-saxonne). $BF$ est le flux sanguin dans le volume de tissu (*Blood Flow* selon une terminologie anglo-saxonne) et $C_v(t)$ est la concentration de l'agent de contraste dans la veine drainant le volume de tissu (fonction de sortie veineuse ou *Venous Output Function* - VOF - selon une terminologie anglo-saxonne).

En supposant le système dynamique artère/tissu/veine linéaire et invariant dans le temps, on peut écrire $C_v(t)=C_a(t) \otimes h(t)$ où $h(t)$ est la réponse impulsionnelle du système - ou encore fonction densité de probabilité du temps de transit de l'agent de contraste dans le tissu - et $\otimes$ désigne le produit de convolution. Une solution formelle de l'équation différentielle précédente avec condition initiale $C(t=0)=0$ s'écrit alors $C(t)=BF \cdot C_a(t) \otimes R(t)$ où $R(t)$ est la fonction de répartition complémentaire du temps de transit dans le volume de tissu (*residue function* selon une terminologie anglo-

$$R(t) = H(t) - \int_0^t h(\tau)\,d\tau$$

saxonne) définie par où $H$ est la fonction généralisée créneau de Heaviside. A partir de la réponse impulsionnelle et de la fonction de répartition complémentaire, on définit un nouveau paramètre hémodynamique, le temps de transit moyen dans le tissu (*Mean Transit Time* - MTT - selon une terminologie anglo-saxonne) :

$$MTT = \int_0^{+\infty} t.h(t)\,dt = \int_0^{+\infty} R(t)\,dt \quad (\text{si } \lim_{t \to +\infty} t \cdot h(t) = 0 )\ .$$

On peut définir également le volume sanguin dans le volume de tissu (*Blood Volume* ou *BV* selon une terminologie anglo-saxonne) par la relation $BV = BF \cdot MTT$.

Les paramètres hémodynamiques tels que *BF, MTT* ou *BV* ainsi que la fonction de répartition complémentaire $R(t)$ sont actuellement estimés comme suit, dans le cas de l'imagerie de perfusion par résonance magnétique nucléaire.

Pour chaque voxel, le signal de perfusion expérimental $S_{\exp}(t)$ échantillonné aux instants de mesure $t_i, i=1,N$,

$$C(t_i) = -\frac{1}{k \cdot TE} \ln\left[ \frac{S_{\exp}(t_i)}{\widehat{S_0}} \right]\ .$$

est converti en une courbe de concentration $C(t)$ par la relation : $\forall i=1,N$ La constante $k$ est fixée pour tous les voxels à une valeur arbitraire non nulle (e.g. $k \cdot TE = 1$). La constante $S_0$ est estimée en prenant, par exemple, sa moyenne avant que l'agent de contraste n'arrive. Notons que cela n'est possible que si l'acquisition des signaux de perfusion démarre suffisamment tôt par rapport au temps d'arrivée de l'agent de contraste (*Bolus Arrival Time* - BAT - selon une terminologie anglo-saxonne). A partir de la courbe de concentration $C(t)$, et en supposant connue la fonction d'entrée artérielle théorique associée $C_a(t)$, le produit $BF \cdot R(t)$ est estimé par déconvolution numérique.

Diverses approches ont été envisagées pour obtenir les fonctions d'entrée artérielles théoriques $C_a(t)$ afin de déconvoluer les courbes de concentration $C(t)$.

Dans une première approche, une fonction d'entrée artérielle globale expérimentale est choisie manuellement par le praticien. Elle peut être mesurée, par exemple, au niveau de l'artère sylvienne controlatérale ou de l'artère carotide interne pour l'imagerie de perfusion du cerveau, ou obtenue par des mesures supplémentaires, par exemple optiques. Si elle permet d'obtenir des signaux avec hauts rapports signal sur bruit, cette approche a néanmoins de nombreux inconvénients. Tout d'abord, elle nécessite une intervention humaine et/ou des mesures supplémentaires. Ceci n'est pas souhaitable en situation d'urgence clinique et rend les procédures et les résultats finaux plus difficilement reproductibles. Ensuite et surtout, cette fonction d'entrée artérielle globale ne correspond pas aux fonctions d'entrée artérielles locales de chaque voxel. Elle en diffère en termes de délai (car les fonctions d'entrée artérielles locales sont en général en retard par rapport à la fonction d'entrée artérielle globale prise en amont du système vasculaire) et de dispersion (car la propagation de l'agent de contraste est plus lente en aval qu'en amont du système vasculaire). Or on sait que ces phénomènes ont au final une incidence considérable sur les estimations des paramètres hémodynamiques puisque, par symétrie du produit de convolution, ces défauts se répercutent directement dans l'estimation de la fonction de répartition complémentaire. Ainsi, par exemple, on n'obtient pas au final une estimation du véritable temps de transit moyen (*MTT*) entre la fonction d'entrée artérielle locale et la fonction de sortie veineuse locale mais seulement un temps de transit moyen entre la fonction d'entrée artérielle globale et la fonction de sortie veineuse. Afin de pallier ces discordances, certains auteurs ont introduit de nouveaux paramètres descriptifs tels que le paramètre

$$TMAX = \arg\max_t R(t)$$

quantifiant le délai entre la fonction d'entrée artérielle globale et les fonctions d'entrée artérielles locales, quand bien même ils n'interviennent pas dans le modèle de perfusion standard original (dans lequel la fonction d'entrée artérielle est la véritable fonction d'entrée artérielle locale pour chaque voxel). D'autres méthodes tendent à minimiser l'influence de ces discrépances des fonctions d'entrée artérielles locales sur l'estimation des paramètres hémodynamiques. Elles introduisent toutefois de nouvelles inconnues dans le problème global et ne font que l'éluder.

Selon une deuxième approche, une fonction d'entrée artérielle globale est obtenue automatiquement à partir d'images de perfusion via des techniques de traitement du signal telles que le partitionnement de données ou l'analyse en composantes indépendantes (*Independent Component Analysis* - ICA - selon une terminologie anglo-saxonne). Si cette approche permet de s'affranchir de l'intervention humaine, elle ne résout pas les problèmes de délai et de dispersion inhérents aux fonctions d'entrée artérielles globales et introduit de nouvelles inconnues (e.g. il se peut qu'on obtienne des fonctions de sortie veineuses au lieu des fonctions d'entrée artérielles).

Selon une troisième approche, des fonctions d'entrée artérielle locales sont obtenues automatiquement à partir des images de perfusion à l'aide de techniques de traitement du signal et de critères de sélection. On recherche par exemple la « meilleure » fonction dans le voisinage immédiat du voxel tissulaire courant où l'on souhaite estimer les paramètres hémodynamiques ou les fonctions de répartition complémentaires. L'objet de cette troisième approche est d'obtenir au final des estimations moins biaisées et plus précises en s'affranchissant, du moins en partie, des problèmes de délai et de dispersion. Cependant, rien ne garantit *a priori* et *a posteriori* que les fonctions d'entrée artérielle locales ainsi obtenues sont des approximations pertinentes de la « véritable fonction locale » pour le voxel d'intérêt. Par exemple, cette « véritable » fonction pourrait ne pas se situer dans le voisinage considéré (s'il est trop petit) ou, au contraire, pourrait être confondu avec une autre fonction d'entrée artérielle (s'il est trop grand). De plus, cette « meilleure » fonction d'entrée artérielle locale est recherchée parmi des fonctions d'entrée artérielle « normales » (i.e. avec un temps d'arrivée de l'agent de contraste de courte/grande précocité, de grande amplitude, etc.). Or il s'agit précisément de distinguer fonctions d'entrée artérielles normales et fonctions d'entrée artérielles pathologiques, par exemple ischémiques. Par conséquent, même si les résultats finaux peuvent être meilleurs qu'avec une approche globale, les incertitudes sur ces fonctions d'entrée artérielles locales et, *a fortiori,* sur les paramètres hémodynamiques ou les fonctions de répartition complémentaires demeurent en grande partie.

Pour effectuer l'opération de déconvolution de la courbe de concentration expérimentale $C(t)$ par la fonction d'entrée artérielle $C_a(t)$ théorique donnée par les méthodes décrites précédemment, le modèle standard de convolution $C(t)=BF \cdot C_a(t) \otimes R(t)$ est tout d'abord discrétisé temporellement, par exemple, suivant l'approximation de la méthode des rectangles :

$$C(t_i) = BF \cdot \int_0^{t_i} C_a(\tau) \cdot R(t-\tau) d\tau \approx BF \cdot \Delta t \cdot \sum_{k=0}^{i} C_a(t_i) \cdot R(t_i - t_k)$$

$\forall i = 1, N,$ où $\Delta t$ est la période d'échantillonnage. On se ramène alors à un système linéaire $Ad = c$ en posant

$$A = \Delta t. \begin{pmatrix} C_a(t_1) & 0 & \dots & 0 \\ C_a(t_2) & C_a(t_1) & \ddots & \vdots \\ \vdots & \vdots & \ddots & 0 \\ C_a(t_N) & C_a(t_{N-1}) & \dots & C_a(t_1) \end{pmatrix} \quad b = \begin{vmatrix} R(t_1) \\ R(t_2) \\ \dots \\ R(t_N) \end{vmatrix} \quad c = \begin{vmatrix} C(t_1) \\ C(t_2) \\ \dots \\ C(t_N) \end{vmatrix}$$

$$d = BF.b$$

En pratique, la matrice $A$ est très mal conditionnée et presque singulière, de sorte qu'on ne peut pas inverser numériquement ce système linéaire sous peine d'obtenir des solutions dénuées de sens et des estimations aberrantes. Il faut donc recourir à diverses méthodes pour obtenir par exemple une pseudo-inverse $\tilde{A}^{-1}$ de la matrice $A$ et par suite une estimation $\hat{d}$ de $d$ par $\hat{d} = \tilde{A}^{-1}.c$. Parmi ces méthodes d'obtention d'une pseudo-inverse, on peut citer les méthodes basées sur la troncature des valeurs singulières de $A$ (*Truncated Singular Value Decomposition,* - (T)SVD) telle que la méthode sSVD (*Simple Singular Value Decomposition* - selon une terminologie anglo-saxonne), la méthode cSVD (*Circular Singular Value Decomposition* - selon une terminologie anglo-saxonne) et la méthode oSVD (*Oscillation index Singular Value Decomposition* - selon une terminologie anglo-saxonne) ou encore la déconvolution de Hunt dans le domaine fréquentiel.

De manière plus générale, on peut minimiser un critère du type $\|Ad-c\|^2+\|\Gamma d\|^2$ où $\|\Gamma d\|^2$ est un terme de régu-

$$\hat{d} = \arg\min_{d} \left( \|Ad - c\|^2 + \|\Gamma d\|^2 \right).$$

larisation privilégiant certaines solutions et obtenir une estimation de $d$ par Parmi ces méthodes, nous pouvons citer la régularisation de Tikhonov, l'utilisation de la transformée en ondelettes, etc.

Une fois $\hat{d}$ obtenue, on peut obtenir une estimation $\widehat{BF}$ de $BF$ par $\widehat{BF} = \hat{d}(t_1) = \hat{d}(0)$ puisque, par définition,

$\lim_{t \to 0^+} R(t) = 1$. Cependant, on constate que l'on estime souvent $BF$ par $\widehat{BF} = \max_{i=1}^{N} \hat{d}(t_i)$, par exemple dans le cadre des méthodes à base de Décomposition en Valeurs Singulières, afin de compenser les sous-estimations systé-

matiques en $\hat{d}(0)$ inhérents à ces méthodes. Par suite, on obtient une estimation $\hat{b}$ de $b$ par $\hat{b} = \dfrac{\hat{d}}{\widehat{BF}}$ puis une estimation

de $MTT = \displaystyle\int_{0}^{+\infty} R(t)\,dt$ par $\widehat{MTT} = \Delta t \cdot \displaystyle\sum_{i=1}^{N} \hat{b}(t_i)$ en suivant, par exemple, l'approximation par la méthode des rectangles. Enfin, on obtient typiquement une estimation de $BV$ par $\widehat{BV} = \widehat{BF}.\widehat{MTT}$ même si l'estimation d'un produit n'est pas le produit des estimations.

On trouve de nombreuses variantes de ces méthodes à base de fonction(s) d'entrée artérielle(s) : par exemple, les fonctions d'entrée artérielles expérimentales peuvent être préalablement ajustées à un modèle théorique paramétrique ou semi-paramétrique $C_a(t,\Theta_a)$ où $\Theta_a$ est un vecteur de paramètres, afin d'accroître artificiellement le rapport signal sur bruit. Le signal peut être également sur-échantillonné artificiellement afin de rendre la déconvolution numérique plus stable ou encore de s'affranchir des éventuels problèmes dus au phénomène de recirculation, lorsqu'il y a chevauchement temporel du signal de circulation de l'agent de contraste (*first pass*) et du signal de recirculation (*second pass*). Mais ces variantes restent basées sur des méthodes de déconvolution numérique telles que la décomposition en valeurs singulières tronquée.

D'après certaines études comparées, parmi les différentes méthodes testées sur données synthétiques censées simuler des données réelles typiques, la déconvolution par décomposition en valeurs singulières et ses variantes (décomposition en valeurs singulières linéaire tronquée (sSVD), circulaire tronquée (cSVD) ou circulaire tronquée lissée (oSVD)) donnent au final les meilleures estimations des paramètres *BF* et *MTT* en termes de biais (erreur systématique par rapport à la valeur véritable), précision (écart-type de l'estimation par rapport à la valeur véritable en fonction du rapport signal-sur-bruit des signaux d'entrée) et robustesse par rapport aux diverses fonctions de répartition complémentaires $R(t)$ et aux diverses fonctions d'entrée artérielles $C_a(t)$ pouvant se présenter en pratique suivant les patients, les types de tissus, les pathologies, etc.

Cependant, outre les problèmes relatifs à la sélection des fonctions d'entrée artérielles décrits précédemment, cette famille de méthodes numériques souffre de graves problèmes inhérents.

Tout d'abord, les estimations $\hat{d}$ de $BF.b$ ne sont pas décroissantes au cours du temps mais oscillantes, à tel point qu'elles peuvent parfois prendre des valeurs négatives. Or, $R(t)$ qui est la quantité d'agent de contraste restant dans le voxel à un instant $t$, est une fonction nécessairement décroissante et positive. Des méthodes *ad hoc* telles que la oSVD permettent de réduire ces oscillations aberrantes mais elles subsistent car elles sont inhérentes à la décomposition en valeurs singulières. C'est la raison pour laquelle avec cette famille de méthodes, on estime les flux sanguins

*BF* par $\widehat{BF} = \max_{i=1}^{N} \hat{d}(t_i)$ alors que dans le cadre du modèle standard de perfusion, on devrait au contraire les estimer

par $\widehat{BF} = \hat{d}(0)$ car $\lim_{t \to 0^+} R(t) = 1$ . En prenant le maximum au lieu de la valeur à l'origine, on espère seulement gommer l'effet de ces oscillations sur les estimations de *BF*. Celles-ci ne peuvent donc être parfaitement satisfaisantes. En particulier, la précision des estimations des *BF* pouvant être atteinte par des méthodes plus rigoureuses d'estimation des fonctions de répartition complémentaires et donc des paramètres hémodynamiques reste inconnue. Ainsi, ces méthodes de déconvolution numérique entrent en contradiction avec le modèle standard de la perfusion puisqu'elles fournissent des solutions ne respectant pas les propriétés dudit modèle.

Afin de s'affranchir de ce problème et obtenir des estimations de fonctions de répartition complémentaires physiologiquement admissibles, des modèles paramétriques $R(t,\Theta_R)$ pour ces fonctions de répartition complémentaires ont été introduits, $\Theta_R$ étant le vecteur des paramètres dudit modèle. Ces modèles sont ajustés aux signaux expérimentaux, par exemple par la méthode de Bayes. Cependant, cette approche peut apparaître prématurée à ce jour. En effet, il faudrait préalablement disposer d'estimations non-paramétriques des fonctions de répartition complémentaires pour déterminer des modèles paramétriques ou semi-paramétriques adéquats à les décrire car des simulations de Monte-Carlo ont montré que si ces modèles ne sont pas parfaitement adaptés pour décrire correctement toutes les formes de fonctions de répartition complémentaires pouvant se présenter en pratique, alors les estimations résultantes des paramètres hémodynamiques tels que *MTT* ou *BF* deviennent aberrantes. Le choix des modèles théoriques pour les fonctions de répartition complémentaires est donc critique et il ne peut se faire convenablement qu'en confrontant les modèles à des données expérimentales. D'où la nécessité de disposer de méthodes non-paramétriques pour estimer ces fonctions de répartition complémentaires, afin éventuellement de les remplacer dans un second temps par des méthodes paramétriques ou semi-paramétriques classiques permettant d'obtenir des estimations physiologiquement admissibles des fonctions de répartition complémentaires.

Or, comme mentionné précédemment, les estimations obtenues par des méthodes de déconvolution telles que les méthodes à base de décomposition en valeurs singulières sont en contradiction avec la définition même de

$$R(t) = H(t) - \int_0^t h(\tau) d\tau$$

dans le cadre du modèle standard de perfusion. Elles ne sont pas physiologiquement et physiquement admissibles. Il n'est donc pas possible d'ajuster des modèles théoriques paramétriques ou semi-paramétriques à ces estimations. A fortiori, il n'est pas possible de comparer plusieurs modèles et de sélectionner les plus adaptés à décrire les fonctions de répartition expérimentales. Il n'est donc plus possible de progresser dans la modélisation et la compréhension des phénomènes de perfusion à cause des défauts inhérents à de méthodes numériques telles que les méthodes à base de décomposition en valeurs singulières.

Par ailleurs, il s'avère que le problème sous-jacent à l'estimation non-paramétrique des fonctions de répartition complémentaires et des paramètres hémodynamiques n'est pas un « simple » problème de déconvolution de courbes de concentration empiriques par des fonctions d'entrée artérielles empiriques. En effet, ce pourrait être le cas si les fonctions d'entrée artérielles étaient réellement des données du problème, connues avec une certitude absolue et une précision infinie. Or on ne dispose au mieux que des signaux artériels expérimentaux mesurés et donc connus seulement aux bruits de mesure près, qu'il faut de plus convertir préalablement en courbes de concentration. Autrement dit, en supposant les fonctions d'entrée artérielles empiriques mesurées égales aux fonctions d'entrée artérielles théoriques, on néglige les bruits de mesure sur les signaux expérimentaux et les incertitudes provenant de l'estimation ou de la conversion des courbes de concentration à partir de ces signaux.

Le modèle standard de convolution $C(t)=BF\cdot C_a(t)\otimes R(t)$ porte uniquement sur des signaux théoriques qui ne peuvent pas, en général, être mesurés directement. En réalité, les signaux mesurés sont typiquement la somme des signaux théoriques et du bruit de mesure. Ainsi, les signaux de perfusion tissulaire et artériel expérimentaux s'écrivent respectivement $S_{exp}(t)=S_{th}(t)+\xi_t$ et $S_{a_{exp}}(t) = S_{a_{th}}(t) + \xi_t^a$ où $\xi_t$ et $\xi_t^a$ sont des processus stochastiques d'espérances mathématiques nulles modélisant les bruits de mesure. On a de plus $S_{th}(t)=S_0 e^{-C_{th}(t)}$ et $S_{ath}(t)=S_0 e^{-C_{ath}(t)}$ où $C_{th}(t)$ est la courbe de concentration théorique et $C_{ath}(t)$ est la fonction d'entrée artérielle théorique dans le cas de l'imagerie de perfusion par résonance magnétique ou, de manière plus générale, $S_{th}(t)=\Psi(C_{th}(t),\Theta_S)$ comme décrit précédemment. Le modèle standard de perfusion théorique appliqué aux signaux expérimentaux mesurés par imagerie de perfusion doit donc s'écrire $C_{th}(t)=BF\cdot C_{ath}(t)\otimes R(t)$ soit, dans le cas de l'imagerie de perfusion par Résonance magnétique nucléaire :

$$\ln \frac{S_{exp}(t) - \xi_t}{S_0} = BF \cdot \ln \frac{S_{aexp}(t) - \xi_t^a}{S_{0a}} \otimes R(t)$$

ou de manière équivalente :

$$\ln \frac{S_{exp}(t) + \xi_t}{S_0} = BF \cdot \ln \frac{S_{aexp}(t) + \xi_t^a}{S_{0a}} \otimes R(t)$$

non pas :

$$C_{exp}(t) = CBF \cdot \left[ C_{a_{exp}}(t) \right] \otimes R(t) + \xi_t$$

où $C_{exp}(t)$ est la courbe de concentration expérimentale et $C_{a_{exp}}(t)$ est la fonction d'entrée artérielle expérimentale, qui est le modèle mathématique de convolution implicite erroné sur lequel se basent la plupart des méthodes de déconvolution. On voit d'ailleurs qu'il est préférable d'écrire le modèle standard de perfusion sous la forme :

$$\begin{cases} S_{\exp}(t) = S_0 e^{-C_{\mathrm{th}}(t)} + \xi_t \\ S_{\mathrm{aexp}}(t) = S_{0a} e^{-C_{\mathrm{ath}}(t)} + \xi_t^a \\ C_{\mathrm{th}}(t) = BF.C_{\mathrm{ath}}(t) \otimes R(t) \end{cases}$$

afin d'éviter de devoir prendre le logarithme de variables aléatoires qui ne sont pas toujours positives.

Il est connu que les incertitudes de mesure sur le signal à déconvoluer ont une influence considérable sur le résultat final de l'opération de déconvolution : une infime variation sur le signal d'entrée due à ces incertitudes peut engendrer une variation considérable sur le résultat final. C'est d'ailleurs pour pallier ces problèmes et réduire ces instabilités que des méthodes de déconvolution telles que la régularisation de Tikhonov ou les méthodes à base de décomposition en valeurs singulières ont été introduites. A fortiori, l'influence des bruits de mesure et des incertitudes sur les fonctions d'entrée artérielles, aujourd'hui entièrement négligée, est encore plus considérable : le bruit de mesure artériel $\xi_t^a$ et les incertitudes sur $S_{0a}$ interviennent désormais dans la matrice de convolution $A$ et sont donc propagées et amplifiées. Cette négligence des erreurs et des incertitudes sur les fonctions d'entrée artérielle provoque des erreurs importantes sur l'estimation des paramètres hémodynamiques, en même temps qu'une illusion de précision sur ces estimations. Certaines méthodes visent à gommer les bruits de mesure dans les fonctions d'entrée artérielles afin de minimiser ce problème qui reste éludé à ce jour. Il serait préférable de disposer de méthodes permettant de propager les incertitudes sur les fonctions d'entrée artérielles sur l'estimation des paramètres hémodynamiques et des fonctions de répartition complémentaires afin de maîtriser et de quantifier les erreurs d'estimation.

D'autre part, il conviendrait également d'éviter d'écrire le modèle standard de la perfusion comme $C_{th}(t)=BF.C_{ath}(t)\otimes R(t)$. En effet, le modèle standard de la perfusion définit en premier lieu la réponse impulsionnelle $h(t)$ du système dynamique artère/tissu/veine à partir de laquelle la fonction de répartition complémentaire $R(t)$ est calculée

par $$R(t) = H(t) - \int_0^t h(\tau)d\tau \ .$$ Il convient donc d'écrire le modèle standard de la perfusion théorique en fonction de la réponse impulsionnelle $h(t)$ comme $$C_{th}(t) = BF.C_{ath}(t) \otimes \left[ H(t) - \int_0^t h(\tau)d\tau \right],$$ d'ajuster ce modèle afin d'estimer la réponse impulsionnelle $h(t)$ aux instants de mesure $t_j, j=1,N$ par $\hat{h}$ pour obtenir ensuite une estimation $\hat{R}$ de

la fonction de répartition complémentaire par, par exemple, $$\hat{R}(t_j) = \left[ H(t_j) - \Delta t \cdot \sum_{i=2}^j \hat{h}(t_i) \right]$$ (méthode d'approximation des rectangles). En particulier, mieux vaut ne pas estimer la réponse impulsionnelle $h(t)$ à partir de l'estimation de la fonction de répartition complémentaire $\hat{R}$. Du point de vue numérique, on comprend aisément qu'il est préférable d'estimer la dérivée ($h(t)$) pour estimer ensuite la primitive ($R(t)$) plutôt que l'inverse. On constate néanmoins que la fonction de répartition complémentaire $R(t)$ est estimée directement au lieu de la réponse impulsionnelle $h(t)$.

D'autre part, le problème de la déconvolution est mal posé et admet a priori une infinité de solutions possibles. A fortiori, le problème mal posé auquel l'on fait face en imagerie de perfusion afin d'estimer des paramètres hémodynamiques, des réponses impulsionnelles, des fonctions de répartition complémentaires ou des fonctions d'entrée artérielles, est un problème de déconvolution triplé d'un problème de propagation des bruits de mesure et d'un problème de conversion des signaux expérimentaux en courbes de concentration.

Pour tenter de se ramener à un problème bien posé admettant éventuellement une unique solution, on doit rajouter a priori de l'information, des contraintes sur la solution recherchée parmi l'infinité des solutions possibles a priori. C'est la raison pour laquelle, on trouve de nombreuses méthodes de déconvolution et d'estimation dans l'état de la technique, chaque méthode injectant, de manière plus ou moins explicite, ou plus ou moins directe, un type particulier d'information a priori.

Le document XP011248726 (Schmid et al., IEEE Trans. Med. Imaging, vol. 28, pp. 789-798, 2009) illustre un exemple de contraintes ad hoc supplémentaires et non physiologiques dans un processus d'estimation des paramètres hémodynamiques réduisant le champ des solutions possibles a priori.

Ainsi et à titre d'exemple, nous pouvons citer la régularisation de Tikhonov classique telle que décrite précédemment. Dans sa version la plus courante, la matrice $\Gamma$ vaut $\Gamma = \alpha I_N$. Ceci pénalise les solutions de grande norme euclidienne, le scalaire $\alpha$ quantifiant le poids de cette pénalisation, de cette contrainte.

Pour sa part, les méthodes à base de troncature des valeurs singulières, classique en imagerie de perfusion, consiste à annuler les valeurs singulières de la matrice de convolution plus petites qu'un certain seuil qui joue donc le rôle de paramètre de régularisation. Ces petites valeurs singulières sont reliées aux composantes du signal de plus hautes fréquences, de sorte que la troncature des valeurs singulières agit comme un filtre passe-bas. Or d'une part, les fonctions de répartition complémentaires sont des signaux à bande infinie du fait de la discontinuité en $t=0$ (on rappelle que les fonctions de répartition complémentaire sont définies sur $]-\infty,+\infty[$. D'autre part, la troncature des petites valeurs singulières ne correspond pas directement et seulement à un ajout d'information de nature physiologique mais surtout à un ajout d'information de nature purement algébrique, en l'occurrence l'appartenance à un sous-espace vectoriel de dimension donnée. On constate d'ailleurs que des oscillations de trop hautes fréquences demeurent. En particulier, les signaux théoriques estimés en reconvoluant les fonctions de répartition complémentaire par les fonctions d'entrée artérielles ont tendance à suivre les bruits de mesure (phénomène de surajustement ou overfitting - suivant une terminologie anglo-saxonne). De plus, le seuil de troncature ne correspondant pas au poids d'une contrainte physiologique explicite (mais seulement à forcer les solutions à appartenir à un certain sous-espace vectoriel), son effet est indirect et complexe et il est difficile de donner des critères généraux pour déterminer ses valeurs les plus appropriées suivant les différents types de signaux. Réciproquement, il n'est pas garanti que l'on puisse convenablement optimiser un tel critère, par exemple la rugosité de la fonction de répartition complémentaire dans le cas de la méthode oSVD car l'action de la troncature des valeurs singulières sur le critère est très indirecte.

Les méthodes actuelles d'estimation de paramètres hémodynamiques ou de fonction de répartition complémentaires ne permettent pas en outre d'obtenir une estimation de la précision sur ces estimations (i.e. écart-type de l'estimateur) et encore moins de quantifier la confiance qu'on peut accorder aux estimations et aux estimations de la précision sur ces estimations. En particulier, il est difficile de quantifier la qualité de l'ajustement (*Goodness of fit,* selon une terminologie anglo-saxonne) du modèle standard de la perfusion par les méthodes de déconvolution et d'estimation. On peut constater ainsi que les méthodes d'estimation à base de troncature des valeurs singulières ont tendance à sur-ajuster (*overfitting,* selon une terminologie anglo-saxonne) les signaux expérimentaux. Les signaux estimés ne sont pas lisses mais, au contraire, ont tendance à suivre le bruit de mesure. On peut donc par conséquent obtenir des valeurs faibles de mesures de la qualité de l'ajustement des modèles telle que la statistique classique du $\chi^2$ (*Sum of Squared Errors,* selon une terminologie anglo-saxonne). Ces valeurs faibles incitent à penser que l'ajustement est de bonne qualité alors qu'il ne l'est pas du fait du surajustement. Ces statistiques sont donc dans ce cas inadaptées et trompeuses et devraient être écartée si on souhaite pouvoir détecter et prendre en compte les phénomènes de surajustement. Il n'est donc pas évident d'introduire de « bonnes » mesures de la qualité de l'ajustement pour les méthodes de déconvolution et d'estimation des paramètres hémodynamiques suivant l'état de la technique. Il est donc souhaitable de disposer de méthodes pour lesquelles une telle mesure d'ajustement est définie de manière univoque, permet de prendre en compte le sur-ajustement et de quantifier seulement l'adéquation des données au modèle standard de la perfusion.

En résumé, les méthodes actuelles d'estimation des paramètres hémodynamiques, des réponses impulsionnelles ou des fonctions de répartition complémentaires sont empreintes de nombreuses erreurs méthodologiques et de nombreuses approximations non maîtrisées. L'interprétation des résultats est ainsi souvent rendue difficile et l'imagerie de perfusion n'est pas pleinement exploitée.

L'objet de la présente invention est de répondre à l'ensemble des inconvénients engendrés par l'utilisation des méthodes connues. L'invention a pour principal objet de fournir de nouvelles méthodes permettant de rechercher l'estimation $\hat{h}$ de la réponse impulsionnelle $h$ parmi des solutions vérifiant certaines contraintes de nature physiologique et hémodynamique, sans pour autant introduire de contraintes *ad hoc* non forcement vérifiées et surtout invérifiables par l'expérience ou de nature non physiologique ou hémodynamique. De plus, ces méthodes permettent de déterminer les poids de telles contraintes de manière automatique et univoque, sans avoir à recourir à des méthodes *ad hoc.*

L'invention permet ainsi de prétendre que le problème de l'estimation des paramètres hémodynamiques, des fonctions de répartition complémentaires ou des fonctions d'entrée artérielles, est à présent bien posé. L'invention permet d'y apporter la seule et unique solution (éventuellement multiple).

Parmi les principaux avantages conférés par l'invention, nous pouvons citer, de manière non limitative, le fait de pouvoir :

- traduire quantitativement des informations physiologiques qualitatives ou semi-qualitatives douces sur les paramètres hémodynamiques, les réponses impulsionnelles, les fonctions de répartition complémentaires ou les fonctions d'entrée artérielles ;
- prendre en compte explicitement les incertitudes et les erreurs sur les fonctions d'entrée artérielles et sur les signaux expérimentaux et, les propager sur les estimations des quantités d'intérêt ;
- améliorer l'estimation desdits paramètres, des réponses impulsionnelles, des fonctions de répartition complémentaires ou des fonctions d'entrée artérielles en termes de biais (erreur systématique), de précision (erreur statistique), de linéarité et de robustesse vis-à-vis des différentes configurations pouvant se présenter dans la pratique ;
- obtenir des intervalles de confiance - et même des paris sur lesdits intervalles de confiance - sur les estimations des paramètres hémodynamiques, des réponses impulsionnelles, des fonctions de répartition complémentaires ou des fonctions d'entrée artérielles, pour préciser et augmenter la confiance que l'on peut accorder à ces estimations ;
- obtenir des estimations non-paramétriques des réponses impulsionnelles, des fonctions de répartition complémentaires ou des fonctions d'entrée artérielles admissibles du point de vue physiologique et hémodynamique et compatible avec le modèle standard de la perfusion, pour permettre dans une étape ultérieure l'ajustement, la comparaison et enfin la sélection de modèles théoriques paramétriques ou semi-paramétriques pour lesdites réponses impulsionnelles, lesdites fonctions de répartition complémentaires ou lesdites fonctions d'entrée artérielles ;
- quantifier dans quelle mesure les fonctions d'entrée artérielles en entrée peuvent être effectivement des fonctions d'entrée artérielles admissibles pour le tissu dans chaque voxel d'intérêt ;
- fournir des mesures objectives et quantitatives de l'adéquation d'un modèle de perfusion global, permettant au final la comparaison et la sélection des modèles de perfusion globaux les plus appropriés.

A cette fin, il est prévu un procédé tel que défini par la revendication 1 pour estimer une quantité d'intérêt parmi une pluralité d'un système dynamique artère/tissu/veine d'un volume élémentaire - dit voxel - d'un organe. Un tel procédé est destiné à être mis en oeuvre par une unité de traitement d'un système d'analyse d'imagerie de perfusion et comporte une étape pour estimer ladite quantité d'intérêt à partir d'une donnée de perfusion expérimentale. Pour rechercher ladite quantité d'intérêt estimée parmi des solutions vérifiant certaines contraintes de nature physiologique et hémodynamique, sans pour autant introduire des contraintes ad hoc non forcement vérifiées et surtout invérifiables par l'expérience ou de nature non physiologique ou hémodynamique, l'étape pour estimer selon l'invention, consiste à évaluer - suivant une méthode de Bayes - une distribution marginale a posteriori pour ladite quantité d'intérêt par :

- l'assignation d'une distribution de probabilité directe de la donnée de perfusion sachant les paramètres intervenant dans l'estimation des quantités d'intérêt du système dynamique artère/tissu/veine du voxel considéré ;
- l'assignation d'une distribution de probabilité conjointe *a priori* desdites quantités, par l'introduction d'une information strictement douce sur la réponse impulsionnelle dudit système dynamique et par l'application du Principe du Maximum d'Entropie sur ladite réponse impulsionnelle dudit système dynamique afin d'obtenir la distribution de probabilité *a priori* de ladite réponse impulsionnelle dudit système dynamique.

L'invention prévoit en outre, un procédé tel que défini par la revendication 2 pour estimer une quantité d'intérêt parmi une pluralité d'un système dynamique artère/tissu/veine d'un volume élémentaire - dit voxel - d'un organe, ledit système dynamique étant linéaire, invariant dans le temps et déterminé formellement par la relation $C(t)=BF \cdot C_a(t) \otimes R(t)$ où $C(t)$ est la concentration d'un agent de contraste circulant dans un voxel, $C_a(t)$ est la concentration dudit agent de contraste dans l'artère alimentant ledit voxel, $BF$ est le flux sanguin dans ledit voxel, $\otimes$ désigne le produit de convolution et $R(t)$ est la fonction de répartition complémentaire du temps de transit dans ledit voxel. Un tel procédé est destiné à être mis en oeuvre par une unité de traitement d'un système d'analyse d'imagerie de perfusion, et comportant une étape pour estimer ladite quantité d'intérêt à partir d'une donnée de perfusion expérimentale. Selon l'invention, et tout comme précédemment, ladite étape pour estimer consiste à évaluer, suivant une méthode de Bayes, une distribution marginale a posteriori pour ladite quantité d'intérêt par :

- l'assignation d'une distribution de probabilité directe de la donnée de perfusion sachant les paramètres intervenant dans l'estimation des quantités d'intérêt du système dynamique artère/tissu/veine du voxel considéré ;
- l'assignation d'une distribution de probabilité conjointe *a priori* desdites quantités, par l'introduction d'une information strictement douce sur la fonction de répartition complémentaire du temps de transit $R(t)$ dans ledit voxel et par l'application du Principe du Maximum d'Entropie sur R(t) afin d'obtenir la distribution de probabilité *a priori* de R(t).

De manière préférée, l'invention prévoit en outre que dans les deux cas, l'assignation d'une distribution de probabilité conjointe *a priori* desdites quantités peut être réalisée par l'introduction d'une information strictement douce sur la courbe de concentration dudit agent de contraste dans l'artère alimentant le voxel et par l'application du Principe du Maximum d'Entropie sur ladite courbe de concentration dudit agent de contraste afin d'obtenir la distribution de probabilité *a priori* de ladite courbe de concentration dudit agent de contraste. Avantageusement, un procédé conforme à l'invention peut être est mis en oeuvre par itérations successives pour une pluralité de voxels considérés.

Pour estimer la précision sur l'estimation d'une quantité d'intérêt, un procédé conforme à l'invention peut comporter une étape pour calculer une information complémentaire sous la forme d'un intervalle de confiance associé à une quantité d'intérêt estimée.

Selon un mode de réalisation préféré, un tel procédé peut en outre comporter une étape pour calculer une information complémentaire sous la forme d'un taux de pari sur un intervalle de confiance associé à une quantité d'intérêt estimée.

Il peut en outre être prévu qu'un procédé conforme à l'invention puisse comporter une étape pour calculer une information complémentaire sous la forme d'une mesure de l'adéquation du produit de :

- l'assignation de la distribution de probabilité directe de la donnée de perfusion sachant les paramètres intervenant dans le problème de l'estimation des quantités d'intérêt du système dynamique artère/tissu/veine du voxel considéré ;
- l'assignation de la distribution de probabilité conjointe a priori desdites quantités.

Pour délivrer toute estimation, un procédé conforme à l'invention peut comporter une étape pour délivrer une quantité d'intérêt estimée à une interface homme-machine apte à la restituer à un utilisateur.

Selon qu'une information complémentaire est calculée, un tel procédé peut comporter en outre une étape pour délivrer toute information complémentaire associée à ladite quantité d'intérêt estimée, à une interface homme-machine apte à la restituer à un utilisateur.

Selon un mode d'application préféré, l'invention prévoit que la donnée de perfusion expérimentale puisse consister en un vecteur de valeurs d'un signal de perfusion expérimental ou en la conversion de ce dernier en un vecteur de valeurs d'une courbe de concentration.

L'invention concerne en outre - selon un deuxième objet - une unité de traitement comportant des moyens de mémorisation, des moyens pour communiquer avec le monde extérieur et des moyens de traitement. Les moyens pour communiquer sont aptes à recevoir du monde extérieur une donnée de perfusion expérimentale. Les moyens de traitement sont quant à eux adaptés pour mettre en oeuvre un procédé pour estimer une quantité d'intérêt parmi une pluralité d'un système dynamique artère/tissu/veine d'un volume élémentaire - dit voxel - d'un organe, conformément à l'invention.

Pour délivrer à un utilisateur une estimation élaborée selon un procédé conforme à l'invention, les moyens pour communiquer d'une telle unité de traitement peuvent délivrer une quantité d'intérêt estimée selon un format approprié à une interface homme-machine apte à la restituer à un utilisateur.

Selon ce mode de réalisation, les moyens pour communiquer peuvent en outre délivrer une information complémentaire associée à une quantité d'intérêt estimée selon un format approprié à une interface homme-machine apte à la restituer à un utilisateur.

Selon un troisième objet, l'invention concerne en outre, un système d'analyse d'imagerie de perfusion comportant une unité de traitement conforme à l'invention et une interface homme-machine apte à restituer à un utilisateur une quantité estimée selon un procédé conforme également à l'invention et mis en oeuvre par ladite unité de traitement.

D'autres caractéristiques et avantages apparaîtront plus clairement à la lecture de la description qui suit et à l'examen des figures qui l'accompagnent parmi lesquelles :

- les figures 1 et 2 présentent deux variantes de réalisation d'un système d'analyse d'imagerie de perfusion ;
- Les figures 3 et 4 présentent respectivement une image de perfusion, obtenue par un appareil d'imagerie par Résonance Magnétique Nucléaire, d'une tranche d'un cerveau humain avant l'injection d'un agent de contraste et pendant la circulation de celui-ci dans les tissus dudit cerveau ;
- les figures 5a et 5b présentent un signal de perfusion $S(t)$ par Résonance Magnétique Nucléaire typique relatif à un voxel d'un cerveau humain ;
- la figure 6 présente une courbe de concentration $C(t)$ typique d'un agent de contraste circulant au sein d'un voxel d'un cerveau humain;
- la figure 7 présente une fonction d'entrée artérielle $C_a(t)$ typique ;
- la figure 8 présente un procédé conforme à l'invention ;
- la figure 9 présente une carte relative à des volumes sanguins cérébraux estimés conformément à l'invention ;
- la figure 10 présente une carte relative à des flux sanguins cérébraux - en cas d'ischémie cérébrale - estimés conformément à l'invention ;
- la figure 11 présente une carte relative à des temps de transit moyen estimés conformément à l'invention ;
- la figure 12 concerne une carte relative à une probabilité qu'un flux sanguin cérébral soit dans un intervalle de confiance.

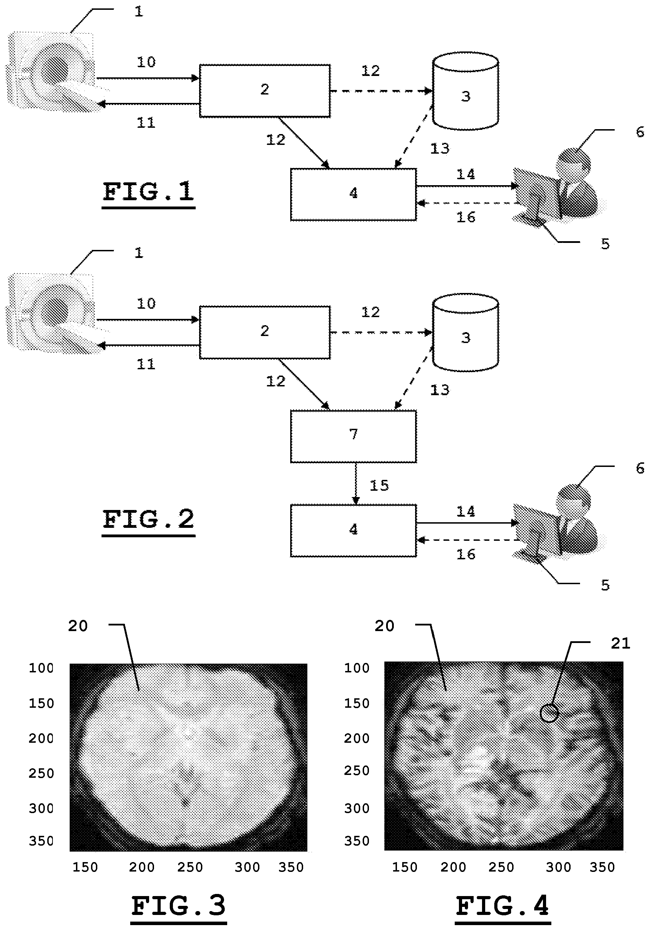

La figure 1 permet de présenter un système d'analyse d'images de perfusion. Un appareil 1 d'imagerie par Résonance Magnétique Nucléaire ou par Tomodensitométrie est commandé à l'aide d'une console 2. Un utilisateur peut ainsi choisir des paramètres 11 pour piloter l'appareil 1. A partir d'informations 10 produites par l'appareil 1, on obtient une pluralité de séquences d'images numériques 12 d'une partie d'un corps d'un humain ou d'un animal. A titre d'exemple préféré, nous illustrerons les solutions issues de l'art antérieur ainsi que l'invention à l'aide d'images numériques issues de l'observation d'un cerveau humain. D'autres organes pourraient aussi être considérés.

Les séquences d'images 12 peuvent optionnellement être stockées au sein d'un serveur 3 et constituer un dossier médical 13 d'un patient. Un tel dossier 13 peut comprendre des images de différents types, telles que des images de perfusion ou de diffusion. Les séquences d'images 12 sont analysées au moyen d'une unité de traitement 4 dédiée. Ladite unité de traitement comporte des moyens pour communiquer avec le monde extérieur pour recueillir les images. Lesdits moyens pour communiquer permettent en outre à l'unité de traitement de délivrer in fine à un praticien 6 ou à un chercheur, une estimation de paramètres hémodynamiques 14 à partir des images de perfusion 12, au moyen d'une interface homme-machine adaptée 5. L'utilisateur 6 du système d'analyse peut ainsi confirmer ou infirmer un diagnostic, décider une action thérapeutique qu'il jugera adéquate, approfondir des travaux de recherche... Optionnellement, cet utilisateur peut paramétrer le fonctionnement de l'unité de traitement 4 au moyen de paramètres 16. Par exemple, il peut ainsi définir des seuils d'affichage ou choisir les paramètres estimés qu'il souhaite visualiser.

La figure 2 illustre une variante de réalisation d'un système d'analyse pour lequel une unité de prétraitement 7 analyse des séquences d'images 12 pour en déduire des données de perfusion 15 par voxel. L'unité de traitement 4 chargée d'estimer les paramètres hémodynamiques 14 est ainsi déchargée de cette action et met en oeuvre un procédé d'estimation à partir de données de perfusion 15 réceptionnés par ses moyens pour communiquer avec le monde extérieur.

La figure 3 illustre un exemple d'image typique 12 d'une tranche de 5 millimètres d'épaisseur d'un cerveau humain. Cette image est obtenue par Résonance Magnétique Nucléaire. A l'aide de cette technique, on peut obtenir, pour chaque tranche, une matrice de 128 x 128 voxels dont les dimensions sont de 1,5 x 1,5 x 5 millimètres. A l'aide d'une interpolation bilinéaire on peut produire une image à plat de 458 x 458 pixels telle que l'image 20.

La figure 4 illustre une image 20 similaire à celle présentée en liaison avec la figure 3. Toutefois cette image est obtenue après une injection d'un agent de contraste. Cette image est un exemple d'image de perfusion typique d'un cerveau. Les artères apparaissent ainsi clairement contrairement à la même image décrite en figure 3. Selon des techniques connues, il est possible de choisir une ou plusieurs fonctions d'entrée artérielle 21 dans l'hémisphère controlatérale à l'hémisphère pathologique pour estimer des paramètres hémodynamiques.

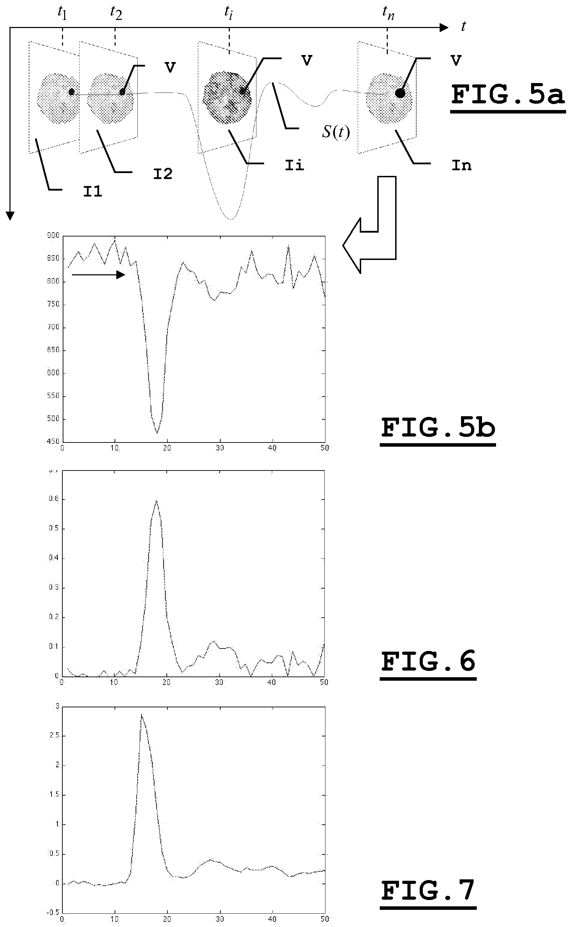

La figure 5b permet d'illustrer un exemple de signal de perfusion $S(t)$ par Résonance Magnétique Nucléaire tel que les données 15 délivrés par l'unité de prétraitement 7 décrite en liaison avec la figure 2. Le signal de perfusion est ainsi représentatif de l'évolution d'un voxel au cours du temps t à la suite d'une injection d'un agent de contraste. A titre d'exemple, la figure 5b décrit un tel signal sur une durée de 50 secondes. L'axe des ordonnées décrit l'intensité du signal dont l'unité est arbitraire. Pour obtenir un tel signal, l'unité de traitement 4 selon la figure 1 (ou en variante l'unité de prétraitement 7 selon la figure 2), analyse une séquence de n images de perfusion par Résonance Magnétique Nucléaire I1, I2, ..., Ii, ..., In à des instants $t_1$, $t_2$, ..., $t_i$, ..., $t_n$ comme le décrit, à titre d'exemple, la figure 5a. Pour un voxel donné, par exemple pour le voxel V, on détermine un signal de perfusion $S(t)$ représentatif de l'évolution du voxel au cours du temps $t$ à la suite d'une injection d'un agent de contraste.

La figure 6 présente une courbe de concentration déduite d'un signal de perfusion tel que celui-ci décrit en figure 5b. Comme déjà évoqué précédemment, il existe une relation entre un signal de perfusion et une courbe de concentration associée. Ainsi, en Imagerie de Perfusion par Résonance Magnétique Nucléaire, il existe une relation exponentielle $S(t)=S_0.e^{-k.TE.C(t)}$ où $S_0$ est l'intensité moyenne du signal avant l'arrivée de l'agent de contraste, $TE$ est le temps d'écho (*echo time*) et $k$ est une constante dépendant de la relation entre la susceptibilité paramagnétique et la concentration de l'agent de contraste dans le tissu.

La figure 6 permet ainsi de visualiser au cours du temps, l'évolution de la concentration d'un agent de contraste au sein d'un voxel. On note un pic de forte amplitude lors du premier passage (*first pass*, en langue anglaise) de l'agent de contraste dans le voxel suivi de pics d'amplitudes plus faibles lié à un phénomène de recirculation (*second pass*, en langue anglaise) dudit agent de contraste.

La figure 7 illustre quant à elle, une fonction d'entrée artérielle typique $C_a(t)$ représentative de la circulation d'un agent de contraste au sein d'un voxel artériel tel que le voxel 21 présenté en liaison avec la figure 4. La figure 7 permet de constater notamment que le phénomène de recirculation après un premier passage de l'agent de contraste est très faible.

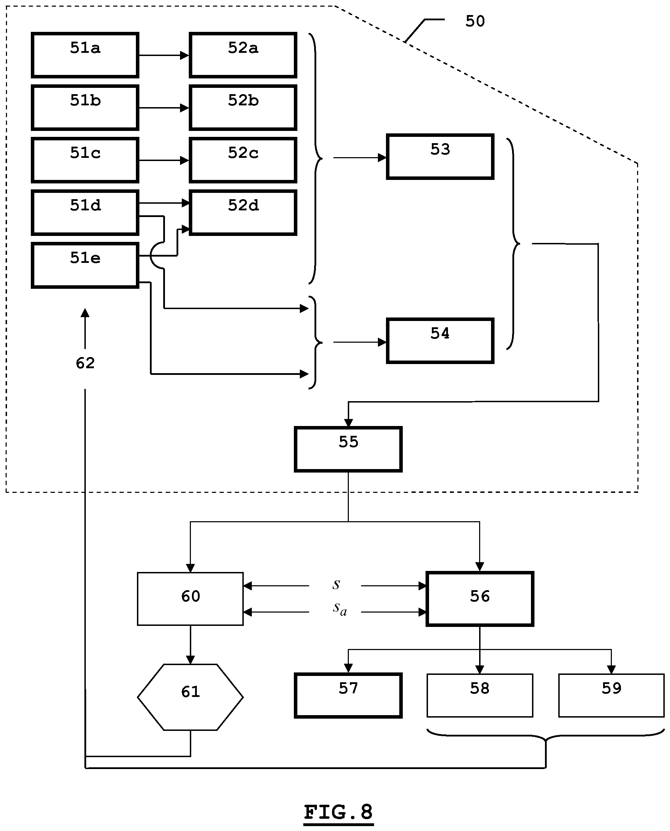

La figure 8 décrit un exemple de procédé - conforme à l'invention - pour estimer une quantité d'intérêt parmi une pluralité d'un système dynamique artère/tissu/veine d'un voxel d'un organe. Un tel procédé peut être mis en oeuvre par une unité de traitement d'un système d'analyse d'imagerie de perfusion tel que le système décrit en liaison avec les figures 1 ou 2 et adapté en conséquence.

Un procédé conforme à l'invention comporte principalement une étape 56 pour assigner une ou plusieurs distributions marginales a posteriori de différentes quantités d'intérêt que l'on cherche à estimer, telles que des paramètres hémodynamiques, les valeurs de la réponse impulsionnelle théorique aux instants de mesure ou celles de la fonction de répartition complémentaire. Il comporte en outre une étape 57 pour calculer ladite estimation.

Pour assigner une telle distribution marginale a posteriori, il est nécessaire de configurer 50 l'unité de traitement. Cette configuration peut être réalisée de préférence par l'unité de traitement elle-même, à partir d'un ou plusieurs paramètres de configuration. La configuration peut également se traduire par la constitution d'une bibliothèque d'une ou plusieurs distributions marginales a posteriori, bibliothèque préétablie et mémorisée dans une mémoire de programme de ladite unité. L'invention prévoit que ladite bibliothèque peut être enrichie au fur et à mesure de son utilisation voire délivrée par une unité externe de calcul apte à réaliser ladite configuration à partir du ou des paramètres de configuration et apte à coopérer avec l'unité de traitement pour délivrer ladite bibliothèque.

Un procédé conforme à l'invention peut comporter ainsi des étapes de configuration mises en oeuvre préalablement à l'assignation 56, parmi lesquelles sont nécessaires et suffisantes :

- l'assignation 54 de la distribution directe de probabilité des signaux expérimentaux sachant tous les paramètres intervenant dans le problème de l'estimation des quantités d'intérêt du système dynamique artère/tissu/veine du voxel considéré ;
- l'assignation 53 de la distribution de probabilité conjointe a priori de tous ces paramètres.

L'invention permet d'estimer une ou plusieurs quantités d'intérêt dans des cas d'application divers :

- les fonctions d'entrée artérielles théoriques sont supposées connues avec une certitude absolue et une précision infinie ;
- les fonctions d'entrée artérielles locales diffèrent de la fonction d'entrée artérielle globale donnée notamment par un délai temporel inconnu qu'il s'agira d'estimer également ;
- le signal d'entrée artériel est seulement mesuré, éventuellement à un délai temporel près ;
- les fonctions d'entrée artérielles ne sont pas données et les signaux d'entrée artériels ne sont pas mesurés - cas d'espèce a priori le plus proche du réel.

Les étapes de configuration peuvent dépendre du cas d'application considéré.

La figure 8 permet d'illustrer un procédé conforme à l'invention selon un premier exemple d'application pour lequel un signal d'entrée artériel est mesuré, éventuellement à un délai temporel $\tau$ près.

Un modèle de perfusion expérimental $M$ peut s'écrire comme :

$$M : \begin{cases} s = \Psi\left(c, \Theta_S\right) + \xi \\ s_a = \Psi\left(a, \Theta_{S_a}\right) + \xi_a \\ c = BF \cdot Ab\left(t - \tau\right) = BF \cdot B\left(t - \tau\right)a \end{cases}$$

$b = [R(t_1),...,R(t_N)]^T$ est un vecteur des valeurs de la fonction de répartition complémentaire théorique inconnue.

Comme expliqué précédemment, ce vecteur peut être éventuellement exprimé à partir du vecteur des valeurs

$$b = \left[1, 1, 1 - \Delta t \cdot h\left(t_2\right),..., 1 - \Delta t \cdot \sum_{i=1}^{N-1} h\left(t_N\right)\right]^T$$

de la réponse impulsionnelle $h = [h(t_1) = 0,...,h(t_N)]^T$. Par exemple,

$$b = \left[1, 1 - \Delta t \cdot h\left(t_2\right),..., 1 - \Delta t \cdot \sum_{i=2}^{N} h\left(t_N\right)\right]^T$$

en suivant la méthode d'approximation des rectangles à gauche ou
en suivant la méthode d'approximation des rectangles à droite ;

$c = [C(t_1),...,C(t_N)]^T$ est un vecteur des valeurs de la concentration théorique inconnue de l'agent de contraste dans le voxel ;

$s = [S(t_1),...,S(t_N)]^T$ est un vecteur de mesures expérimentales du signal d'intensité par imagerie de perfusion, réel ou complexe ;

$\Theta_S$ est un vecteur des paramètres reliant $S(t)$ à $C(t)$ ;

$\xi = [\xi(t_1),...,\xi(t_N)]^T$ est un vecteur des bruits de mesures sur ledit signal mesuré ;

$s_a = [S_a(t_1),...,S_a(t_N)]^T$ est un vecteur des mesures du signal d'entrée artériel expérimental $S_a(t)$ ;

$\Theta_{S_a}$ est un vecteur des paramètres reliant $S_a(t)$ à $C_a(t)$ ;

$\xi_a = [\xi_a(t_1),...,\xi_a(t_N)]^T$ est un vecteur des bruits de mesures sur ledit signal artériel ;

$a = [C_a(t_1),...,C_a(t_N)]^T$ est un vecteur des valeurs de la fonction d'entrée artérielle théorique $C_a(t)$ inconnue ; $A$ est une matrice de convolution associée au vecteur des valeurs de la fonction d'entrée artérielle théorique inconnue $a$ obtenue en approchant numériquement l'intégrale de convolution par la méthode d'approximation des rectangles, des trapèzes, ou par des méthodes d'ordre supérieur telles que les méthodes de Simpson, de Boole, de Gauss-Legendre, etc. $A$ peut être également une matrice de convolution circulante, telles que celles utilisées dans le cadre des méthodes de déconvolution par troncature des valeurs singulières cSVD et oSVD.

Nous noterons $\Theta$ le vecteur des paramètres hémodynamiques du modèle $M$, ici par exemple $\Theta=(BF,\tau)$.

En liaison avec les figures 1, 2 et 8, un procédé mis en oeuvre par une unité de traitement 4 peut comporter deux premières étapes de configuration 51d et 51e consistant à introduire respectivement une information $I_S$ sur le vecteur des mesures du signal expérimental $s$ et une information $I_{S_a}$ sur le vecteur des mesures du signal d'entrée artériel expérimental $s_a$.

Si l'on s'en tient, par exemple, seulement aux deux premiers moments $E(\xi,\xi_a)\equiv(0,0)$ et $E\left(\xi^2,\xi_a^2\right)=\left(\sigma_S,\sigma_{S_a}\right)$ du couple des vecteurs des bruits de mesure réels correspondant, alors le Principe du Maximum d'Entropie (entropie de Shannon différentielle sous la mesure de référence de Lebesgue) requiert que les vecteurs $\xi$ et $\xi_a$ doivent être regardés comme des processus stochastiques mutuellement indépendants, blancs, stationnaires Gaussiens et d'écarts-types respectifs $\sigma_S$ et $\sigma_{S_a}$.

De manière plus générale, nous noterons $(E_S,E_{S_a})$ les paramètres caractérisant le couple des vecteurs des bruits de mesure $(\xi,\xi_a)$. Par exemple $(E_S,E_{S_a})=(\sigma_S,\sigma_{S_a})$.

Un procédé conforme à l'invention comporte une étape de configuration 54 pour assigner une distribution de probabilité conjointe directe du couple des vecteurs des mesures $(s,s_a)$ sachant les vecteurs $a$ et $b$, le vecteur $\Theta$, les vecteurs de paramètres $\Theta_S$ et $\Theta_{S_a}$ et enfin le couple des vecteurs $(E_S,E_{S_a})$. Ladite distribution de probabilité conjointe directe s'écrit alors $p(s,s_a|a,b,\Theta,\Theta_S,\Theta_{S_a},E_S,E_{S_a},I_S,I_{S_a},M)$.

Par exemple, nous avons :

$$p\left(s,s_a\big|a,b,\Theta,\Theta_S,\Theta_{S_a},\sigma_S,\sigma_{S_a},I_S,I_{S_a},M\right)\propto$$

$$\left(\sigma\sigma_a\right)^{-N}\exp\left\{\sum_{i=1}^{N}\frac{\left[S(t_i)-\Psi\left(BF.Ab(t_i-\tau),\Theta_S\right)\right]^2}{2\sigma^2}+\frac{\left[S_a(t_i)-\Psi\left(a(t_i),\Theta_{S_a}\right)\right]^2}{2\sigma_a^2}\right\}$$

si on regarde $\xi$ et $\xi_a$ comme des processus stochastiques mutuellement indépendants, blancs, stationnaires Gaussiens et d'écarts-types respectifs $\sigma_S$ et $\sigma_{S_a}$. L'invention permet en variante, de pouvoir exprimer différemment ladite distribution de probabilité conjointe directe dans le cas où l'on s'intéresse, non pas directement au vecteur $b$ des valeurs de la fonction de répartition complémentaire théorique inconnue, mais au vecteur des valeurs $h$ de la réponse impulsionnelle. Pour cela, il suffit d'exprimer $b$ en fonction de $h$. La distribution de probabilité conjointe directe 54 s'écrit alors par exemple

$$p\left(s,s_a\big|a,h,\Theta,\Theta_S,\Theta_{S_a},\sigma_S,\sigma_{S_a},I_S,I_{S_a},M\right)\propto$$

$$\left(\sigma\sigma_a\right)^{-N}\exp\left\{\sum_{i=1}^{N}\frac{\left[S(t_i)-\Psi\left(BF.Ab(t_i-\tau),\Theta_S\right)\right]^2}{2\sigma^2}+\frac{\left[S_a(t_i)-\Psi\left(a(t_i),\Theta_{S_a}\right)\right]^2}{2\sigma_a^2}\right\}$$

Nous considèrerons par la suite, sans perte de généralité, que l'on s'intéresse préférentiellement au vecteur $h$ pour les raisons exposées précédemment.

On pourrait en outre assigner de la même manière la distribution de probabilité conjointe directe du couple des vecteurs des mesures de signaux complexes, en multipliant les distributions de probabilité directes de leurs parties réelles et imaginaires.

L'invention prévoit également une variante lorsque les vecteurs des valeurs des signaux de perfusion $s$ et $s_a$ peuvent être convertis précisément en des vecteurs de valeurs de courbes de concentration $c_{exp}$, par exemple par

$$c_{\exp}\left(t_i\right) = -\frac{1}{k \cdot TE} \ln \frac{s\left(t_i\right)}{\widehat{S}_0},$$

$i=1,N$ en imagerie de perfusion par Résonance Magnétique Nucléaire. Il est possible de procéder ainsi, par exemple, lorsque les intensités moyennes $S_0$ des signaux de perfusion par résonance magnétique nucléaire avant l'arrivée de l'agent de contraste peuvent être estimées avec précision et indépendamment des autres paramètres. Selon cette variante, on peut montrer qu'il est encore légitime d'assigner une distribution de probabilité gaussienne pour le couple des vecteurs $(c_{\exp}, c_{\exp a})$ sachant tous les paramètres telle que :

$$p\left(c_{\exp}, c_{\exp a} \middle| a, h, \Theta, \sigma, \sigma_a, I_S, I_{S_a}, M\right) \propto$$

$$\left(\sigma\sigma_a\right)^{-N} \exp\left\{\sum_{i=1}^{N} \frac{\left[c_{\exp}\left(t_i\right) - BF.Ab\left(t_i\right)\right]^2}{2\sigma^2} + \frac{\left[c_{\exp a}\left(t_i\right) - a\left(t_i\right)\right]^2}{2\sigma_a^2}\right\}$$

$\sigma$ et $\sigma_a$ sont désormais les écarts-types des bruits de mesure sur $c_{\exp}$ et $c_{\exp a}$ respectivement.

Plus généralement, nous utiliserons le terme « donnée de perfusion expérimentale » pour désigner indifféremment un vecteur de valeurs d'un signal de perfusion expérimental $s$ ou sa conversion en un vecteur des valeurs d'une courbe de concentration $c_{\exp}$. Dans la suite nous notons, sans perte de généralité, $s$ et $s_a$ les données de perfusion expérimentales.

Par ailleurs, le procédé comporte trois étapes de configuration 51a, 51b et 51c consistant à introduire respectivement une information $I_\Theta$ sur les paramètres hémodynamiques $\Theta$ du modèle $M$, une information $I_h$ sur la réponse impulsionnelle $h$ et une information $I_a$ sur la fonction d'entrée artérielle $a$. Les informations introduites aux étapes 51a à 51e constituent, au sens de l'invention, des paramètres de configuration pour configurer - c'est-à-dire - rendre apte une unité de traitement à assigner 56 une distribution marginale a posteriori et ainsi estimer 57 une quantité d'intérêt. A partir de ces informations - ou paramètres de configuration, un procédé conforme à l'invention peut comporter une étape 53 pour assigner une distribution de probabilité conjointe a priori qui s'exprime sous la forme $p(a,h,\Theta,\Theta_{S_a},E_S,E_{S_a}|I_\Theta,I_h,I_a,I_S,I_{S_a},M)$.

En appliquant le Principe du Maximum d'Entropie, cette distribution peut typiquement se factoriser en :

$$p\left(a, h, \Theta, \Theta_S, \Theta_{S_a}, E_S, E_{S_a} \middle| I_\Theta, I_h, I_a, I_S, I_{S_a}, M\right)$$

$$= p\left(\Theta \middle| I_\Theta, M\right) \cdot p\left(h, E_S \middle| I_h, I_S, M\right) \cdot p\left(a, E_{S_a} \middle| I_a, I_{S_a}, M\right) \cdot p\left(\Theta_S, \Theta_{S_a} \middle| I_S, I_{S_a}, M\right)$$

Un tel procédé comporte ainsi une étape 52a consistant à assigner la distribution de probabilité a priori $p(\Theta|I_\Theta,M)$.

A titre d'exemples, on peut assigner une distribution a priori non-informative. Par exemple, si l'information $I_\Theta$ consiste seulement à savoir que $BF$ et $\tau$ appartiennent aux intervalles respectifs $[BF_{\min},BF_{\max}]$ et $[\tau_{\min},\tau_{\max}]$, alors la distribution de probabilité a priori $p(\Theta|I_\Theta,M)$ peut être une distribution a priori de la forme :

$$p\left(\Theta \middle| I_\Theta, M\right) = p\left(BF, \tau \middle| I_\Theta, M\right) =$$

$$\chi_{\left[BF_{\min}, BF_{\max}\right]}\left(BF\right) \cdot \chi_{\left[\tau_{\min}, \tau_{\max}\right]}\left(\tau\right) / \left[\log\left(BF_{\max}\right) - \log\left(BF_{\min}\right)\right] / \left(\tau_{\max} - \tau_{\min}\right) / BF$$

A l'autre extrémité, on peut également assigner des distributions de probabilité informatives telles que les distributions des fréquences relatives empiriques ou les distributions de probabilité marginales a posteriori obtenues à partir d'expériences antérieures, par exemple à partir de techniques d'imagerie de perfusion quantitatives telles que la Tomographie par Emission de Positrons (*Positron Emission Tomography,* PET suivant une terminologie anglo-saxonne) ou l'ASL (Arterial Spin Labelling).

Un procédé conforme à l'invention comporte en outre une étape de configuration 52b consistant à assigner la distribution de probabilité a priori $p(h,E_S|I_h,I_S,M)=p(h|E_S,I_h,M) \cdot p(E_S|I_S,M)$ à partir de l'information $I_h$ (ou $I_b$ si on s'intéresse seulement à la fonction de répartition complémentaire). Cette information est constituée d'informations dures et douces.

Au sens de l'invention, on distingue une information dure d'une information douce. Une information dure correspond à toute proposition Booléenne considérée comme certaine - c'est-à-dire dont la probabilité vaut 1. Par exemple les propositions Booléennes telles que « cette courbe est lisse » et « ce signal suit ce modèle » constituent des informations dures. Par opposition, une information douce concerne toute proposition Booléenne qui consiste à indiquer, et à seulement indiquer avec une certaine probabilité de telles propositions Booléennes. Au final, cela revient à introduire des propositions booléennes telles que « cette courbe est plus ou moins lisse » ou « ce signal suit plus ou moins ce modèle ». Dans la suite, on appelle « information strictement douce » toute information douce qui n'est pas dure.

Afin de bien montrer la différence entre information dure et information douce, considérons le cas le plus simple et le plus extrême. Supposons qu'on veuille estimer une grandeur réelle $x$ à partir d'échantillons $x_1,...,x_n$ indépendants et identiquement distribués suivant une loi normale $N(x,\sigma^2)$. Fixons un nombre réel $a$. Considérons l'information a priori quantitative dure « $x=a$ ». Cette information dure se traduit par la distribution de probabilité a priori de Dirac $p(x|a)=\delta(x-a)$, c'est-à-dire $p(x|a)=1$ si $x=a$ et $p(x|a)=0$ si $x{\neq}a$.

Considérons à présent l'information quantitative douce correspondante : « $x$ est plus ou moins proche de $a$ », c'est-à-dire « $\|x-a\|^2=(x-a)^2$ est plus ou moins grand » où $\|\ \|$ est la norme euclidienne.

Par application du Principe du Maximum d'Entropie, cette information quantitative douce se traduit en la distribution de probabilité a priori normale :

$$p\left(x\middle|a,\varepsilon\right)=\frac{1}{\sqrt{2\pi}\varepsilon}\exp\left[-\frac{\left(x-a\right)^2}{2\varepsilon^2}\right] \quad \text{avec} \quad \varepsilon>0$$

On constate donc que l'information dure et l'information douce correspondante se traduisent par des distributions de probabilité a priori différentes. En particulier, dans la distribution a priori pour l'information douce, il apparaît un nouveau (hyper)paramètre $\varepsilon$ permettant de traduire a priori, de manière quantitative, le « plus ou moins » par l'assignation d'une distribution de probabilité a priori $p(\varepsilon)$. En particulier, on retrouve l'information dure comme cas limite de l'information douce en faisant tendre $\varepsilon$ vers 0. Toutefois, l'invention ne concerne que des informations strictement douces pour lesquelles $\varepsilon{>}0$.

Les distributions a priori dures et strictement douces correspondantes étant toujours différentes, on obtient de manière générale au final des estimations des quantités d'intérêt elles-mêmes différentes. Dans notre exemple extrême, il n'y a en fait pas besoin d'estimer $x$ dans le cas dur puisque l'on sait déjà que $x=a$. En revanche, dans le cas doux, on est ramené à un problème classique d'estimation de l'espérance mathématique d'une loi normale.

Considérons à présent - à titre de deuxième exemple - l'information quantitative dure classique « $y$ réel suit un modèle (semi-)paramétrique $M(x,\Theta)$ », $\Theta$ étant le vecteur des paramètres de $M$ et $y$ étant réel. Par définition, cette information quantitative se traduit en la distribution de probabilité a priori de Dirac

$$p\left(y\middle|x,\Theta,M\right)=\delta\left[y-M\left(x,\Theta\right)\right]$$

Si maintenant $z$ est une donnée expérimentale bruitée telle que $z \sim N(y,\sigma^2)$ alors on a la fonction de vraisemblance ou distribution de probabilité directe

$$p\left(z\middle|x,\Theta,\sigma,M\right)=\frac{1}{\sqrt{2\pi}\sigma}\exp\left\{-\frac{\left[z-M\left(x,\Theta\right)\right]^2}{2\sigma^2}\right\}$$

à partir de laquelle on peut estimer les quantités d'intérêt $\Theta$ et $\sigma$ en appliquant par exemple la règle de Bayes

$$p\left(\Theta,\sigma\middle|z,x,M\right)\propto p\left(\Theta,\sigma\middle|M\right)p\left(z\middle|x,\Theta,\sigma,M\right)$$

Considérons maintenant l'information quantitative douce correspondante « $y$ suit plus ou moins un modèle (semi-)paramétrique $M(x,\Theta)$ ». Cette information douce se traduit en :

« $\|y-M(x,\Theta)\|^2=[y-M(x,\Theta)]^2$ est plus ou moins grand »

Par application du Principe du Maximum d'Entropie, cette information se traduit en la distribution de probabilité a priori

$$p\left(y\middle|x,\varepsilon,\Theta,M\right)=\frac{1}{\sqrt{2\pi}\varepsilon}\exp\left\{-\frac{\left[y-M\left(x,\Theta\right)\right]^{2}}{2\varepsilon^{2}}\right\} \quad \text{avec}\ \ \varepsilon>0$$

Si $z\sim N(y,\sigma^2)$ comme précédemment, on a la fonction de vraisemblance ou distribution de probabilité directe

$$p\left(z\middle|x,\varepsilon,\Theta,\sigma,M\right)=\int p\left(z,y\middle|x,\varepsilon,\Theta,\sigma,M\right)dy=$$

$$\int p\left(z\middle|y,\sigma,M\right)p\left(y\middle|x,\varepsilon,\Theta,M\right)dy=$$

$$\int\frac{1}{\sqrt{2\pi}\sigma}\exp\left[-\frac{\left(z-y\right)^{2}}{2\sigma^{2}}\right]\frac{1}{\sqrt{2\pi}\varepsilon}\exp\left\{-\frac{\left[y-M\left(x,\Theta\right)\right]^{2}}{2\varepsilon^{2}}\right\}dy$$

à partir de laquelle on peut estimer les quantités d'intérêt en appliquant par exemple la règle de Bayes :

$$p\left(\varepsilon,\Theta,\sigma\middle|z,x,M\right)\propto p\left(\varepsilon,\Theta,\sigma\middle|M\right)p\left(z\middle|x,\varepsilon,\Theta,\sigma,M\right)$$

Nous pouvons constater - à travers ce deuxième exemple - que, les informations dures et strictement douces correspondantes peuvent se distinguer non seulement au niveau des distributions de probabilité a priori, comme dans le premier exemple, mais également au niveau des fonctions de vraisemblance. A nouveau, on obtient au final de manière générale des estimations des quantités d'intérêt différentes. Cet exemple montre ainsi comment on peut, de manière générale, « adoucir » des modèles (semi-)paramétriques et des méthodes dures d'estimation de paramètres hémodynamiques au sens de l'invention.

Prenons un troisième exemple afin d'illustrer une information qualitative. Soit $S(t)$ un signal réel et $s=[S(t_1),...,S(t_N)]$ un vecteur de ses valeurs. Considérons l'information qualitative dure « $S(t)$ est lisse ». Cette information qualitative se convertit canoniquement en l'information quantitative « $\left\|\frac{d^{2}S}{dt^{2}}\right\|_{2}^{2}=0$ » où $\|\|\|_{2}$ est la norme fonctionnelle $L^2$. Il s'en suit que $S(t)$ est linéaire ou encore qu'il existe $(a,b)$ tel que $S(t)=at+b$. Il n'existe effectivement rien de plus lisse qu'une droite. Cette information dure se traduit donc au final en la distribution de probabilité a priori $p(S(t)|t,a,b)=\delta[S(t)-at-b]$. En pratique, on est donc ramené à estimer le couple $(a,b)$.

Considérons maintenant l'information qualitative strictement douce correspondante « $S(t)$ est plus ou moins lisse ». Celle-ci se traduit par l'information quantitative strictement douce « $\left\|\frac{d^{2}S}{dt^{2}}\right\|_{2}^{2}$ est plus ou moins grande ». Etant dans un espace fonctionnel, il semble qu'on ne peut pas appliquer directement le Principe du Maximum d'Entropie afin d'obtenir un processus stochastique a priori traduisant cette information douce. En revanche, on peut considérer la version discrétisée temporellement de l'information quantitative « $\left\|\frac{d^{2}s}{dt^{2}}\right\|^{2}$ est plus ou moins grande » ou $\frac{d^{2}s}{dt^{2}}$ est obtenue à partir de $s$ par un schéma d'approximation numérique aux différences finies de type $\frac{d^{2}s}{dt^{2}}=Ds$ de la dérivée seconde.

$$\left\| \frac{d^2 s}{dt^2} \right\|^2 = \left( Ds \right)^T \left( Ds \right) = s^T \left( D^T D \right) s \equiv s^T \Sigma s$$

On a donc

Une application du Principe du Maximum d'Entropie fournit alors la distribution de probabilité a priori normale multivariée pour le vecteur $s$ :

$$p\left( s \mid \varepsilon \right) \propto \exp\left( -\frac{s^T \Sigma s}{2\varepsilon^2} \right)$$

On peut donc mesurer encore la différence entre information dure et information strictement douce. Dans le cas d'une information douce, les $s_i$ sont contraints à être sur une droite $y=at+b$ alors que dans le cas d'une information strictement douce, ils s'en éloignent plus ou moins suivant la valeur de $\varepsilon{>}0$ qu'il s'agit d'estimer. Le premier cas se ramène à un problème d'estimation de deux paramètres $(a,b)$ alors que le second cas se ramène à un problème d'estimation de $N{+}1$ paramètres ($N$ paramètres pour le vecteur $s$ plus l'hyperparamètre $\varepsilon$).

Les notions d'information douce et strictement douce selon l'invention étant clairement définies, revenons aux informations dures et douces sur la réponse impulsionnelle $h(t)$.

$$\int_0^{+\infty} h(t)dt \cong \Delta t \cdot \sum_{i=2}^{N} h\left( t_i \right) = 1$$

Les informations quantitatives dures incluent $h(t_1)=h(0)=0$ et par exemple suivant la méthode des rectangles à droite. Ces deux informations permettent de réduire à $N{-}2$ le nombre des valeurs/paramètres à estimer. Comme nous allons le voir, un choix judicieux peut consister à conserver les valeurs

$$h\left( t_N \right) = 1 / \Delta t - \sum_{i=2}^{N-1} h\left( t_i \right) .$$

$h'=[h(t_2),...,h(t_{N-1})]^T$ et à exprimer $h(t_N)$ par                D'autre part, nous pouvons également considérer l'information quantitative dure $\forall i=1,N\ h(t_i){\geq}0$. Il reste donc à assigner la distribution de probabilité conjointe a priori du vecteur $h'$ en combinant ces informations quantitatives dures avec une information purement qualitative et strictement douce. Considérons - comme évoqué précédemment - par exemple l'information qualitative strictement douce $I_h^1$ « $h(t)$ *est plus ou moins lisse* ». Comme vu précédemment, cette information qualitative $I_h^1$ peut se traduire d'abord en l'information quantitative strictement douce « $\left\| \frac{d^2 h}{dt^2} \right\|_2^2$ *est plus ou moins grand* ».

Après discrétisation temporelle aux instants de mesure $t_i{=}1,N$, une approximation numérique d'ordre 2 de la dérivée seconde de $h$ est donnée par exemple par :

$$\forall i = 2, N-1, \quad \frac{d^2 h\left( t_i \right)}{dt^2} = \frac{h\left( t_{i-1} \right) + h\left( t_{i+1} \right) - 2h\left( t_i \right)}{\Delta t^2} + O\left( \Delta t^2 \right)$$

On peut également utiliser des approximations numériques d'ordre supérieur, par exemple la formule d'ordre 4 : $\forall i=3,N{-}2,$

$$\frac{d^2 h\left( t_i \right)}{dt^2} = \frac{-\frac{1}{12} h\left( t_{i-2} \right) + \frac{4}{3} h\left( t_{i-1} \right) - \frac{5}{2} h\left( t_i \right) + \frac{4}{3} h\left( t_{i+1} \right) - \frac{1}{12} h\left( t_{i+2} \right)}{\Delta t^2} + O\left( \Delta t^4 \right)$$

Ces approximations numériques peuvent s'écrire sous une forme matricielle $\dfrac{d^2 h'}{dt^2} \simeq Dh$ où $D$ est par exemple

$$D = \frac{1}{\Delta t^2} \begin{pmatrix} 0 & 0 & 0 & 0 & \cdots & \cdots & \cdots & 0 \\ 1 & -2 & 1 & 0 & \ddots & & & \vdots \\ 0 & 1 & -2 & 1 & 0 & \ddots & & \vdots \\ 0 & \ddots & \ddots & \ddots & \ddots & \ddots & \ddots & \vdots \\ \vdots & \ddots & \ddots & \ddots & \ddots & \ddots & \ddots & 0 \\ \vdots & & \ddots & 0 & 1 & -2 & 1 & 0 \\ \vdots & & & \ddots & 0 & 1 & -2 & 1 \\ 0 & \cdots & \cdots & \cdots & 0 & 0 & 0 & 0 \end{pmatrix}$$

la matrice carrée                                                                         de dimension $N$ dans le cas de l'approximation d'ordre 2 et $h'$ est tel que défini précédemment.

Le carré de la norme euclidienne de la dérivée seconde de $h'$ peut s'écrire alors

$$\left\| \frac{d^2 h'}{dt^2} \right\|^2 = \left(Dh\right)^T \left(Dh\right) = h^T \left(D^T D\right) h \equiv h^T W_1 h \,.$$

Supposer qualitativement la réponse impulsionnelle $h(t)$ plus ou moins lisse peut donc se traduire de manière

$$\left\| \frac{d^2 h'}{dt^2} \right\| = \sqrt{h^T W_1 h}$$

quantitative par supposer que                                                est plus ou moins grande. On cherche donc la distribution de probabilité a priori $p(h'|h(t_1),h(t_N),I)$ - les points extrêmes $h(t_1)$ et $h(t_N)$ devant être traités séparément - parmi toutes les distributions de probabilité continues de même norme euclidienne $\left\| \dfrac{d^2 h'}{dt^2} \right\|$. En appliquant alors le Principe du Maximum d'Entropie - qui consiste à choisir parmi toutes ces distributions à support l'hyper-quadrant $[0,+\infty]^{N-2}$ celle ayant la plus grande entropie de Shannon différentielle (sous la mesure de référence de Lebesgue) car c'est la plus incertaine et donc la plus honnête - on obtient la distribution conditionnelle gaussienne multivariée tronquée sur $[0,+\infty]^{N-2}$ d'espérance mathématique vectorielle constante M=$(\mu_1,...,\mu_1)^T$ et de matrice variance-covariance $\dfrac{\varepsilon_1^2}{\sigma^2} W_1^{-1}$ (ou la gaussienne multivariée tronquée sur $[0,1]^{N-2}$ pour le vecteur des valeurs de la fonction de répartition complémentaire) :

$$p\left(h'|h(t_1),h(t_N),\mu_1,\varepsilon_1,E_S,I_h^1,M\right) = C_1\left(\mu_1,\varepsilon_1,\sigma_S\right) \exp\left\{ -\frac{\varepsilon_1^2\left(h-M\right)^T W_1 \left(h-M\right)}{2\sigma_S^2} \right\}$$

où $C_1(\mu_1,\varepsilon_1,\sigma_S)$ est la constante de normalisation

$$C_1\left(\mu_1,\varepsilon_1,\sigma_S\right) = \left[ \int_{h'\in[0,+\infty]^{N-2}} \exp\left\{ -\frac{\varepsilon_1^2\left(h-M\right)^T W_1 \left(h-M\right)}{2\sigma_S^2} \right\} dh' \right]^{-1}$$

Il apparaît donc deux nouveaux hyperparamètres dans notre modèle global de perfusion, l'espérance mathé-

matique scalaire $\mu_1$ et la variance fractionnaire inverse $\varepsilon_1$ qui joue le rôle de paramètre de régularisation pour notre modèle de perfusion. $\varepsilon_1$ quantifie la douceur de l'information a priori qualitative douce $I_h^1$ par rapport à l'information quantitative dure procurée par les données expérimentales $s$ et $s_a$.

D'autre part, par définition :

$$p\left(h(t_1), h(t_N) \middle| \mathrm{E}_S, I_h^1, M\right) = p\left(h(t_1) \middle| \mathrm{E}_S, I_h^1, M\right) p\left(h(t_N) \middle| \mathrm{E}_S, I_h^1, M\right) =$$

$$\delta\left[h(t_1)\right] \cdot \delta\left[h(t_N) - 1/\Delta t \cdot \sum_{i=2}^{N-1} h(t_i)\right]$$

Cependant, afin de tenir compte du fait que le délai $\tau$ n'est presque jamais égal à un instant de mesure $t_i, i=1, N$, on pourra poser

$$p\left(h(t_1) \middle| \mu_{1,1}, \varepsilon_{1,1}, \mathrm{E}_S, I_h^1, M\right) = C_{1,1}\left(\mu_{1,1}, \varepsilon_{1,1}, \sigma_S\right) \exp\left\{-\frac{\varepsilon_{1,1}^2 \left[h(t_1) - \mu_{1,1}\right]^2}{2\sigma_S^2}\right\}$$

où $h(t_1) \in [0,1]$ et $C_{1,1}(\mu_{1,1}, \varepsilon_{1,1}, \sigma_S)$ est la constante de normalisation

$$C_{1,1}\left(\mu_{1,1}, \varepsilon_{1,1}, \sigma_S\right) = \left[\int_{h(t_1) \in [0,1]} \exp\left\{-\frac{\varepsilon_{1,1}^2 \left[h(t_1) - \mu_{1,1}\right]^2}{2\sigma_S^2}\right\} dh(t_1)\right]^{-1}$$

de sorte à indiquer et seulement indiquer que $h(\tau) \cong 0^+$.

Au final la distribution de probabilité a priori de $h$ à support l'hyper-quadrant $[0, +\infty]^N$ pour l'information $I_h^1$ peut s'écrire :

$$p\left(h \middle| \mu_1, \varepsilon_1, \mathrm{E}_S, I_h^1, M\right) =$$

$$\delta\left[h(t_1)\right] \delta\left[h(t_N) - 1/\Delta t + \sum_{i=2}^{N-1} h(t_i)\right] C_1\left(\mu_1, \varepsilon_1, \sigma_S\right) \exp\left\{-\frac{\varepsilon_1^2 (h - \mathrm{M})^T W_1 (h - \mathrm{M})}{2\sigma_S^2}\right\}$$

De manière générale, nous noterons $\mathrm{E}_h$ (ou $\mathrm{E}_b$) le vecteur des hyperparamètres de la distribution a priori pour $h$, par exemple $\mathrm{E}_h = (\mu_1, \varepsilon_1)$ ou $\mathrm{E}_h = (\mu_1, \varepsilon_1, \mu_{1,1}, \varepsilon_{1,1})$.

Il reste encore à assigner une distribution de probabilité a priori pour $\mathrm{E}_h$. On peut assigner par exemple la distribution non-informative impropre de Bayes-Laplace-Lhoste-Jeffreys $p\left(\mu_1, \varepsilon_1 \middle| I_h^1, M\right) \propto \varepsilon_1^{-1}$ ou $p\left(\mu_1, \varepsilon_1, \mu_{1,1}, \varepsilon_{1,1} \middle| I_h^1, M\right) \propto \varepsilon_1^{-1} \varepsilon_{1,1}^{-1}$ .

De la même manière, on peut introduire l'information quantitative strictement douce $I_h^2 \ll \left\|\frac{dh}{dt}\right\|_2^2$ *est plus*

$$I_h^k, \quad k = 1, 2, \ldots \quad \ll \quad \left\| \frac{d^k h}{dt^k} \right\|_2^2$$

*ou moins grand* » et de manière plus générale les informations strictement douces *est plus ou moins grand* ».

On obtient ainsi au final de nouvelles distributions de probabilité a priori gaussiennes multivariées tronquées pour *h* par application du Principe du Maximum d'Entropie.

Il convient de remarquer que les informations douces $I_h^1$, $I_h^2$, $\cdots$ telles que décrites précédemment sont les seules informations strictement douces, les seule contraintes qu'il est pleinement légitime d'introduire dans notre problème : ce ne sont pas des hypothèses arbitraires qui peuvent être vérifiées ou pas par l'expérience mais, au contraire, il s'agit juste de l'expression de propriétés physiologiques fondamentales sans lesquelles le problème de l'estimation des paramètres hémodynamiques, des réponses impulsionnelles ou des fonctions de répartition complémentaires n'aurait en fait aucun sens : elles sont logiquement nécessaires et suffisantes à notre problème. Toute autre information serait à l'inverse une simple hypothèse potentiellement vérifiable par l'expérience.

Conformément à l'invention, on peut toutefois introduire d'autres informations strictement douces qui seront de simples hypothèses de travail. Par exemple, on peut indiquer et seulement indiquer que la réponse impulsionnelle suit plus ou moins une certaine forme fonctionnelle sans pour autant la contraindre - à l'aide d'une information dure - à suivre exactement cette forme. L'ajout de ce type d'informations semi-quantitatives et strictement douces permet de déterminer dans quelle mesure les réponses impulsionnelles peuvent être décrites par les formes fonctionnelles proposées. Supposons donc par exemple qu'on veuille introduire l'information strictement douce - telle qu'évoquée préalablement - $I_h^3$ « $h(t)$ *suit plus ou moins un modèle paramétrique ou semi-paramétrique $M_h$:f(t,$\Theta_h$)* », $\Theta_h$ étant le vecteur des paramètres dudit modèle. Comme dit précédemment, de tels modèles paramétriques ont été proposés, par exemple le modèle Γ à deux paramètres donné par :

$$M_h : \begin{cases} f(t, \Theta_h) = t^{\frac{MTT}{\beta} - 1} e^{-t/\beta} / \beta^{\frac{MTT}{\beta}} / \Gamma\left(\frac{MTT}{\beta}\right) \quad MTT > 0, \beta > 0 \\ \\ \Theta_h = (MTT, \beta) \\ \\ \Gamma(\ ) : \text{fonction Gamma d'Euler} \end{cases}$$

Notons que dans ce cas, le paramètre *MTT* peut être estimé directement, sans avoir à estimer numériquement le premier moment de la réponse impulsionnelle (ou l'intégrale de la fonction de répartition complémentaire) comme décrit précédemment.

Indiquer que *h* suit plus ou moins une certaine forme fonctionnelle *f(t,$\Theta_h$)* revient alors à indiquer quantitativement

$$\left\| h - f(t, \Theta_h) \right\|^2 = \sum_{i=1}^{N} \left[ h(t_i) - f(t_i, \Theta_h) \right]^2$$

que la norme euclidienne du vecteur des résidus est plus ou moins grande. En appliquant à nouveau le Principe du Maximum d'Entropie, on trouve de la même manière que la distribution de probabilité a priori de *h* est la gaussienne multivariée tronquée sur l'hyper-quadrant $[0,+\infty]^N$

$$p\left(h \mid \mathrm{E}_h, \mathrm{E}_S, \Theta_h, I_h^3, M_h, M\right) = C_3\left(\varepsilon_3, \sigma_S, \Theta_h\right) \exp\left\{ -\frac{\varepsilon_3^2}{2\sigma_S^2} \sum_{i=1}^{N} \left[ h(t_i) - f(t_i, \Theta_h) \right]^2 \right\}$$

où $C_3(\varepsilon_3, \sigma_S, \Theta_h)$ est la constante de normalisation

$$C_3\left(\varepsilon_3,\sigma_S,\Theta_h\right)=\left[\int\limits_{h\in\left[0,+\infty\right]^N}\exp\left\{-\frac{\varepsilon_3}{2\sigma_S^{\,2}}\left\|h-f\left(t,\Theta_h\right)\right\|^2\right\}dh\right]^{-1}.$$

De manière équivalente, on pourrait introduire une information douce $I_h^4$ telle que « $h(t)$ suit *plus ou moins un vecteur de valeurs donné* $\overline{h}=[\overline{h}(t_1),...,\overline{h}(t_N)]^T$ ».

Par application du Principe du Maximum d'Entropie, on obtient alors la distribution de probabilité a priori :

$$p\left(h\big|\mathrm{E}_h,\mathrm{E}_S,I_h^4,M\right)\propto C_4\left(\varepsilon_4,\sigma_S,\overline{h}\right)\exp\left\{-\frac{\varepsilon_4^{\,2}}{2\sigma_S^{\,2}}\sum_{i=1}^{N}\left[h\left(t_i\right)-\overline{h}\left(t_i\right)\right]^2\right\}$$

où $C_4(\varepsilon_4,\sigma,\overline{h})$ est la constante de normalisation

$$C_4\left(\varepsilon_4,\sigma_S,\overline{h}\right)=\left[\int\limits_{h\in\left[0,+\infty\right]^N}\exp\left\{-\frac{\varepsilon_4^{\,2}}{2\sigma_S^{\,2}}\left\|h-\overline{h}\right\|^2\right\}dh\right]^{-1}.$$

L'invention permet également de pouvoir combiner plusieurs informations douces sur la réponse impulsionnelle $h(t)$ (ou la fonction de répartition complémentaire) et leurs distributions de probabilité a priori correspondantes. Ainsi, si $p\left(h\big|\mathrm{E}_h^1,I_h^1,M\right),...,p\left(h\big|\mathrm{E}_h^n,I_h^n,M\right)$ sont $n$ distributions de probabilité a priori traduisant des informations $I_h^n,...,I_h^n$, de paramètres de régularisation respectifs $\mathrm{E}_h^1,...,\mathrm{E}_h^n$ (avec par exemple $\mathrm{E}_h^1=\left(\varepsilon_1,\mu_1\right)$, $\mathrm{E}_h^3=\left(\varepsilon_3,\Theta_h\right)$, etc.), alors une distribution a priori pour $h$ tenant compte de ces $n$ informations peut s'écrire

$$p\left(h\big|\mathrm{E}_h,I_h,M\right)=\prod_{k=1}^{n}p\left(h\big|\mathrm{E}_h^k,I_h^k,M\right)$$ en notant $\mathrm{E}_h=\left(\mathrm{E}_h^1,...,\mathrm{E}_h^n\right)$ et $I_h=I_h^1\wedge...\wedge I_h^n$.

Pour encoder une information $I_a$ sur la fonction d'entrée artérielle locale, un procédé conforme à l'invention comporte en outre une étape 52c consistant à assigner une distribution de probabilité a priori $p(a,E_{S_a}|I_a,I_{S_a},M) = p(a|E_{S_a},I_a,M)\cdot p(E_{S_a}|I_{S_a},M)$. Une telle distribution est assignée de la même manière que celle relative à la réponse impulsionnelle $h$, en introduisant et en combinant des informations dures et/ou douces sur la fonction d'entrée artérielle. Par exemple, on peut spécifier que la fonction d'entrée artérielle est plus ou moins lisse, positive, unimodale, bimodale, nulle à l'origine, asymptotiquement nulle, d'aire donnée ou encore indiquer et seulement indiquer qu'elle suit plus ou moins un certain modèles paramétrique ou semi-paramétrique $C_a(t,\Theta_a)$ où $\Theta_a$ est un vecteur de paramètres, par exemple le modèle « tri-Gamma » à onze paramètres:

$$M_a : \begin{cases} C_a\left(t,\Theta_a\right) = \\ \dfrac{a\left(t-t_0\right)^{\alpha_0-1} e^{-(t-t_0)/\beta_0}}{\beta_0{}^{\alpha_0}\Gamma\left(\alpha_0\right)} + \dfrac{b\left(t-t_1\right)^{\alpha_1-1} e^{-(t-t_1)/\beta_1}}{\beta_1{}^{\alpha_1}\Gamma\left(\alpha_1\right)} + \dfrac{(1-a-b)\left(t-t_2\right)^{\alpha_2-1} e^{-(t-t_2)/\beta_2}}{\beta_2{}^{\alpha_2}\Gamma\left(\alpha_2\right)} \\ \Theta_a = \left(a,b,\alpha_0,\beta_0,t_0,\alpha_1,\beta_1,t_1,\alpha_2,\beta_2,t_2\right) \\ \Gamma(\ ) : \text{fonction Gamma d'Euler} \end{cases}$$

Comme décrit précédemment, on obtient une distribution de probabilité a priori gaussienne

$$p\left(a\big|E_a^1,E_{S_a},\Theta_a,I_a^1,M_a,M\right) = C\left(\varepsilon_a^1,\sigma_{S_a},\Theta_a\right)\exp\left\{-\frac{\left(\varepsilon_a^1\right)^2}{2\sigma_{S_a}{}^2}\sum_{i=1}^{N}\left[a(t_i)-C_a\left(t_i,\Theta_a\right)\right]^2\right\}$$

où $C\left(\varepsilon_a^1,\sigma_{S_a},\Theta_a\right)$ est la constante de normalisation

$$C\left(\varepsilon_a^1,\sigma_{S_a},\Theta_a\right) = \left[\int_{a\in[0,+\infty]^N}\exp\left\{-\frac{\left(\varepsilon_a^1\right)^2\left\|a-C_a\left(t,\Theta_a\right)\right\|^2}{2\sigma_{S_a}{}^2}\right\}da\right]^{-1}.$$

De même, il est possible d'indiquer et de seulement indiquer (sans quoi on se ramène au cas où la fonction d'entrée artérielle est donnée) que le vecteur $a$ suit plus ou moins un vecteur de valeurs donné $\bar{a}=[\bar{a}(t_1),...,\bar{a}(t_N)]^T$ tel une fonction d'entrée artérielle locale : on obtient la distribution de probabilité a priori

$$p\left(a\big|E_a^2,E_{S_a},I_a^2,M\right) = C_a\left(\varepsilon_a^2,\sigma_{S_a},\bar{a}\right)\exp\left\{-\frac{\left(\varepsilon_a^2\right)^2}{2\sigma_{S_a}{}^2}\sum_{i=1}^{N}\left[a(t_i)-\bar{a}(t_i)\right]^2\right\}$$

où $C(\varepsilon_a,\sigma_a,\bar{a})$ est la constante de normalisation

$$C_a\left(\varepsilon_a^2,\sigma_{S_a},\bar{a}\right) = \left[\int_{a\in[0,+\infty]^N}\exp\left\{-\frac{\left(\varepsilon_a^2\right)^2\left\|a-\bar{a}\right\|^2}{2\sigma_{S_a}{}^2}\right\}da\right]^{-1}.$$

Un procédé conforme à l'invention comporte en outre une étape de configuration 52d consistant à assigner une distribution de probabilité a priori $p(E_S,E_{S_a},\Theta_S,\Theta_{S_a}|I_S,I_{S_a},M)$ qui peut typiquement se factoriser en :

$$p\left(\mathrm{E}_S, \mathrm{E}_{S_a}, \Theta_S, \Theta_{S_a} \mid I_S, I_{S_a}, M\right)$$

$$= p\left(\mathrm{E}_S \mid I_S, M\right) \cdot p\left(\mathrm{E}_{S_a} \mid I_{S_a}, M\right) \cdot p\left(\Theta_S \mid I_S, M\right) \cdot p\left(\Theta_{S_a} \mid I_{S_a}, M\right)$$

On a par exemple la distribution de probabilité a priori non-informative :

$$p\left(\mathrm{E}_S, \mathrm{E}_{S_a}, \Theta_S, \Theta_{S_a} \mid I_S, I_{S_a}, M\right)$$

$$= p\left(\sigma_S \mid I_S, M\right) \cdot p\left(\sigma_{S_a} \mid I_{S_a}, M\right) \cdot p\left(S_0 \mid I_S, M\right) \cdot p\left(S_{0_a} \mid I_{S_a}, M\right)$$

$$= \left(\sigma_S \cdot \sigma_{S_a}\right)^{-1} / \left(S_0{}^{\max} - S_0{}^{\min}\right) / \left(S_{0_a}{}^{\max} - S_{0_a}{}^{\min}\right)$$

En tenant compte des différents hyperparamètres introduits dans les étapes 52b à 52d, la distribution de probabilité conjointe a priori 53 peut se réécrirecomme $p(a, E_a, h, E_h, \Theta, \Theta_S, \Theta_{S_a}, E_S, E_{S_a} \mid I_\Theta, I_h, I_a, I_S, I_{S_a}, M)$.

Pour simplifier les expressions suivantes, nous notons $I = (I_\Theta, I_h, I_a, I_S, I_{S_a}, M)$ l'ensemble des informations introduites comme autant de paramètres de configuration de l'unité de traitement.

Etant données une distribution de probabilité directe 54 et une distribution de probabilité conjointe a priori 53 telles que décrites précédemment, on obtient la distribution de probabilité conjointe a posteriori 55 de tous les paramètres en appliquant la règle de Bayes :

$$p\left(a, \mathrm{E}_a, h, \mathrm{E}_h, \Theta, \Theta_S, \Theta_{S_a}, \mathrm{E}_S, \mathrm{E}_{S_a} \mid s, s_a, I\right) =$$

$$\frac{p\left(a, \mathrm{E}_a, h, \mathrm{E}_h, \Theta, \Theta_S, \Theta_{S_a}, \mathrm{E}_S, \mathrm{E}_{S_a} \mid I\right) \cdot p\left(s, s_a \mid a, h, \Theta, \Theta_S, \Theta_{S_a}, \mathrm{E}_S, \mathrm{E}_{S_a}, I\right)}{p\left(s, s_a \mid I\right)} \propto$$

$$p\left(a, \mathrm{E}_a, h, \mathrm{E}_h, \Theta, \Theta_S, \Theta_{S_a}, \mathrm{E}_S, \mathrm{E}_{S_a} \mid I\right) \cdot p\left(s, s_a \mid a, h, \Theta, \Theta_S, \Theta_{S_a}, \mathrm{E}_S, \mathrm{E}_{S_a}, I\right)$$

La configuration préalable étant réalisée, l'invention permet à présent d'estimer une quantité d'intérêt que nous noterons $\theta$ parmi tous les éléments du vecteur $\Xi = (a, E_a, h, E_h, \Theta, \Theta_S, \Theta_{S_a}, E_S, E_{S_a})$. Par exemple, $\theta = BF$ ou $\theta = \tau$, ou bien encore $\theta = h(t_i)$, etc.

Un procédé conforme à l'invention comporte donc une étape 56 consistant à évaluer la distribution marginale

$$p\left(\theta \mid s, s_a, I\right) = \int_{\Xi \backslash \theta} p\left(\Xi \mid s, s_a, I\right) d\left(\Xi \backslash \theta\right) .$$

a posteriori pour $\theta$, soit

A partir de cette distribution marginale a posteriori, on peut obtenir 57 des estimations $\hat{\theta}$ de $\theta$. Par exemple, on obtient l'estimateur de Bayes sous fonction de coût quadratique $L(\theta - \hat{\theta}^Q) = \|\theta - \hat{\theta}^Q\|^2$ où $\|\|$ est la norme euclidienne en prenant

$$\hat{\theta}^Q = \int_\theta \theta \cdot p\left(\theta \mid s, s_a, I\right) d\theta .$$

l'espérance mathématique de cette distribution                    De même, on peut obtenir l'estimateur du maximum a posteriori $\hat{\theta}^P$ (Maximum a posteriori Estimator, MAP suivant une terminologie anglo-saxonne) par

$$\hat{\theta}^P = \arg\max_\theta p\left(\theta \mid s, s_a, I\right) .$$

A titre d'exemple, on peut obtenir la distribution de probabilité marginale a posteriori de la valeur $h(t_i), i = 1, N$ de la réponse impulsionnelle en chaque instant de mesure $t_i$, en marginalisant tous les autres instants :

$$\forall i = 1, N, \quad p\left(h(t_i)\big| s, s_a, I\right) = \int\limits_{\substack{h(t_j) \\ j \neq i}} p\left(h\big| s, s_a, I\right) dh(t_1)...dh(t_{j \neq i})...dh(t_N)$$

et par suite obtenir des estimations de ces valeurs telles que $\hat{h}^Q(t_i)$ ou $\hat{h}^P(t_i)$.

De la même manière, il est également possible d'obtenir 57 une estimation $\hat{h}(x)$ (ou $\hat{R}(x)$) de la valeur de la réponse impulsionnelle $h(x)$ à un instant $x$ quelconque, non forcément confondu avec un instant de mesure $t_i$. Il suffit pour cela d'introduire $h(x)$ dans l'expression des valeurs de la fonction de répartition complémentaire $R(t)$ comme décrit précédemment et de calculer sa distribution marginale de probabilité. Il est même souhaitable d'introduire de tels instants supplémentaires $x_1,...,x_L$ dans le problème d'estimation puisque les approximations numériques des intégrales telles que

$$\int\limits_0^t h(\tau)\,d\tau \qquad \int\limits_0^{+\infty} h(\tau)\,d\tau = 1 \qquad \int\limits_0^t C_a(\tau)\cdot\left[H(t-\tau) - \int\limits_0^{t-\tau} h(\upsilon)\,d\upsilon\right]d\tau$$

ainsi que l'approximation numérique de $\dfrac{d^2 h}{dt^2}$ ou $\dfrac{dh}{dt}$ seront d'autant meilleures que le nombre d'instants pris en compte sera grand. Les estimations résultantes seront donc également d'autant meilleures.

$$MTT = Eh = \int\limits_0^{+\infty} t \cdot h(t)\,dt \text{ ,}$$

Ensuite, puisque $\qquad$ on peut obtenir des estimations de ce paramètre telles que

$$\widehat{MTT}^Q = \Delta t \cdot \sum_{i=2}^N t_i \cdot \hat{h}^Q(t_i)$$

l'estimation « en moyenne » $\qquad$ ou l'estimation la plus probable

$$\widehat{MTT}^P = \Delta t \cdot \sum_{i=2}^N t_i \cdot \hat{h}^P(t_i)$$

en appliquant une méthode d'intégration numérique, par exemple ici, la méthode des rectangles à droite.

En outre, un procédé selon l'invention peut comporter une étape 58 pour obtenir une estimation de la précision sur l'estimation du paramètre $\theta$, voire des intervalles de confiance pour ces estimations. L'invention prévoit qu'un tel procédé puisse comporter en outre une étape 59 pour obtenir des paris sur lesdits intervalles de confiance. Par exemple, la précision sur l'estimation $\hat{\theta}^Q$ peut être quantifiée par la matrice variance-covariance de la distribution marginale a posteriori pour $\theta$ :

$$\hat{\sigma}_{\hat{\theta}}^{Q\,2} = \int\limits_\theta \left(\theta - \hat{\theta}^Q\right)\left(\theta - \hat{\theta}^Q\right)^T p\left(\theta\big| s, s_a, I\right) d\theta$$

On a alors par exemple un (hyper-) intervalle de confiance à « un sigma » pour $\theta J = [\hat{\theta}^Q - diag(\hat{\sigma}_{\hat{\theta}}^Q), \hat{\theta}^Q + diag(\hat{\sigma}_{\hat{\theta}}^Q)]$

$$p_J = \int\limits_{\theta \in J} p\left(\theta\big| s, s_a, I\right) d\theta$$

et la probabilité que $\theta$ appartienne à cet intervalle $\qquad$ ou, de manière équivalente, le taux

de pari $\dfrac{p_J}{1 - p_J}$ (*odds* suivant une terminologie anglo-saxonne).

Il est également possible d'obtenir des estimations, des intervalles de confiance et des paris sur ces intervalles de confiance pour le paramètre $BV = BF \cdot MTT$, le vecteur des valeurs de la fonction de répartition complémentaire $b$, le vecteur des valeurs de la fonction de sortie veineuse $v = Ah$ ou encore le vecteur $c = BF.Ab$ des valeurs de la courbe de concentration théorique car, connaissant la distribution de probabilité conjointe de plusieurs variables aléatoires, on peut calculer la fonction densité de probabilité d'une fonction quelconque en dépendant. Par exemple, par linéarité de l'espérance mathématique, on obtient immédiatement les estimations des valeurs de la fonction de répartition complé-

$$\hat{b}^Q = \left(1, 1 - \Delta t \cdot \hat{h}^Q\left(t_2\right), ..., 1 - \Delta t \cdot \sum_{i=2}^{N} \hat{h}^Q\left(t_N\right)\right)^T$$

mentaire $R(t)$ à partir de celles de la réponse impulsionnelle

$$\hat{b}^P = \left(1, 1 - \Delta t \cdot \hat{h}^P\left(t_2\right), ..., 1 - \Delta t \cdot \sum_{i=2}^{N} \hat{h}^P\left(t_N\right)\right)^T$$

et                                                                                                                          en suivant par exemple la méthode des rectangles à droite. De la même manière, de la distribution de probabilité conjointe $p(c,\Theta S|s,s_a,I)$ de c et $\Theta_s$, on peut déduire également celle du vecteur $s_{th}=[S_{th}(t_1),...,S_{th}(t_N)]^T$ des valeurs du signal de perfusion théorique $S_{th}(t)$ puisque $s_{th}=\Psi(c,\Theta_S)$. A partir de cette distribution, on peut enfin obtenir 57 des estimations $\widehat{s_{th}}$ ainsi que des hyper-intervalles de confiance 58 et des paris 59 sur ces hyper-intervalles suivant une méthode similaire à celle décrite précédemment.

L'invention prévoit que les intervalles de confiances ou paris sur lesdits intervalles de confiance, puisse permettre d'ajuster 62 la configuration de l'unité de traitement. Ainsi, il est possible, de modifier les paramètres de configuration et proposer des estimations de meilleure qualité.

L'invention prévoit en outre, qu'un procédé puisse comporter une étape 60 pour calculer des résidus

$$r\left(t_i\right) = S\left(t_i\right) - \widehat{s_{th}}\left(t_i\right), i = 1, N$$

entre les signaux par imagerie de perfusion théoriques et expérimentaux. L'invention permet alors de calculer diverses statistiques ou distances $D\left(s, \widehat{s_{th}}\right)$ entre ces vecteurs, la plus classique

$$\chi^2 = \sum_{i=1}^{N} r\left(t_i\right)^2$$

étant la somme des carrés des résidus                         (*Sum of Squared Errors,* SSE selon une terminologie anglo-saxonne). Ces diverses statistiques permettent de quantifier l'adéquation du modèle de perfusion aux données expérimentales. On obtient ainsi des « cartes d'erreur » pour le modèle pour chaque voxel d'intérêt. Pour les raisons évoquées précédemment (i.e. problème de sur-ajustement), la quantification de l'adéquation du modèle de perfusion aux données expérimentales $s$ et $s_a$ pourra se faire de manière particulièrement avantageuse en calculant la probabilité des données expérimentales $(s,s_a)$ sachant le modèle de perfusion en chaque voxel d'intérêt :

$$p\left(s, s_a | I\right) = \int_{\Xi} p\left(s, s_a | \Xi, I\right) p\left(\Xi | I\right) d\Xi$$

Dans ce cas, la carte d'erreur serait basée sur $1-p(s,s_a|I)$.

L'invention prévoit également que l'on puisse appliquer 61 divers tests statistiques ou diverses techniques de diagnostic graphiques tels que les courbes de Henri (*Q-Q plot* suivant une terminologie anglo-saxonne) ou les cartes de Poincaré (*return maps* suivant une terminologie anglo-saxonne) afin de vérifier si les résidus $r(t_i)$ sont bien indépendants, identiquement distribués et gaussiens, etc. L'invention prévoit ainsi, par un processus itératif et essais successifs, de pouvoir corriger et affiner 62 le processus 50 de configuration, notamment de corriger et affiner des modèles de perfusion théoriques afin de progresser dans la modélisation, la compréhension et le traitement des phénomènes de perfusion et d'obtenir in fine de meilleures estimations des paramètres hémodynamiques, des réponses impulsionnelles, des fonctions de répartition complémentaires, des fonctions d'entrée artérielles ou encore des fonctions d'entrée veineuses.

En liaison avec la figure 8, nous nous proposons de décrire à présent un procédé conforme à l'invention selon un deuxième exemple d'application pour lequel les fonctions d'entrée artérielles théoriques sont supposées être données avec une certitude absolue et une précision infinie à un délai temporel $\tau$ près.

Un modèle de perfusion expérimental $M$ peut s'écrire alors comme :

$$M : \begin{cases} s = \Psi\left(c, \Theta_S\right) + \xi \\ c = BF \cdot Ab\left(t - \tau\right) = BF \cdot B\left(t - \tau\right)a \end{cases}$$

où toutes les quantités sont telles que définies précédemment sauf $a=[C_a(t_1),...,C_a(t_N)]^T$ qui est le vecteur des valeurs de la fonction d'entrée artérielle théorique désormais supposé connu.

La distribution de probabilité conjointe directe s'écrit alors $p(s|a,h,\Theta,\Theta_S,E_S,I)$ et la distribution de probabilité conjointe a priori $p(h,E_h,\Theta,\Theta_S,E_S|I)$ avec $I=(I_\Theta,I_h,I_S,M)$. Ces distributions s'assignent comme décrit précédemment. La règle de Bayes devient

$$p\left(h, E_h, \Theta, \Theta_S, E_S \middle| s, a, I\right) \propto p\left(h, E_h, \Theta, \Theta_S, E_S \middle| a, I\right) \cdot p\left(s \middle| a, h, \Theta, \Theta_S, E_S, I\right)$$

On obtient ensuite des estimations, des intervalles de confiance et des paris sur ces intervalles de confiance pour tout paramètre $\theta \in \Xi=(h,E_h,\Theta,\Theta_S,E_S)$ de la même manière que celle décrite précédemment.

En liaison avec la figure 8, nous nous proposons de décrire à présent un procédé conforme à l'invention selon un troisième exemple d'application pour lequel les fonctions d'entrée artérielles théoriques ne sont pas données et où les signaux d'entrée artériels ne sont pas mesurés.

Un modèle de perfusion expérimental $M$ peut s'écrire alors comme :

$$M : \begin{cases} s = \Psi\left(c, \Theta_S\right) + \xi \\ c = BF \cdot Ab\left(t\right) = BF \cdot B\left(t\right)a \end{cases}$$

où toutes les quantités sont telles que définies précédemment.

La distribution de probabilité conjointe directe s'écrit toujours $p(s|a,h,\Theta,\Theta_S,E_S,I)$ et la distribution de probabilité conjointe a priori s'écrit désormais $p(a,E_a,h,E_h,\Theta,\Theta_S,E_S|I)$ avec $I=(I_\Theta,I_h,I_a,I_S,M)$. La règle de Bayes devient

$$p\left(a, E_a, h, E_h, \Theta, \Theta_S, E_S \middle| s, I\right) \propto p\left(a, E_a, h, E_h, \Theta, \Theta_S, E_S \middle| I\right) \cdot p\left(s \middle| a, h, \Theta, \Theta_S, E_S, I\right)$$

On obtient ensuite des estimations, des intervalles de confiance et des paris sur ces intervalles de confiance pour tout paramètre $\theta \in \Xi=(a,E_a,h,E_h,\Theta,\Theta_S,E_S)$ de la même manière que celle décrite précédemment.

Il convient de remarquer, quelque soit l'exemple d'application retenu, qu'à l'inverse des méthodes connues, un procédé d'estimation conforme à l'invention est une méthode exacte, dans le sens où elle consiste et consiste seulement à traduire des informations qualitatives, quantitatives ou semi-quantitatives dont on dispose a priori sur les quantités d'intérêt dans la Théorie des Probabilités Bayésienne afin de déterminer univoquement l'information a posteriori sur ces mêmes quantités, procurée par le biais des mesures expérimentales. Aucune hypothèse arbitraire qui pourrait ne pas être vérifiée (si tant est qu'elle puisse l'être) - en particulier sur les réponses impulsionnelles ou les fonctions de répartition complémentaires - n'est nécessaire car l'invention introduit seulement les distributions de probabilités les plus incertaines codifiant les différentes contraintes qualitatives douces logiquement nécessaires et suffisantes pour résoudre le problème.

Il s'en suit que, dans les cas où les fonctions d'entrée artérielles ne sont pas supposées données mais tout au plus mesurées, ces méthodes permettent de tester si les signaux d'entrée artériels proposés peuvent effectivement correspondre aux « véritables » fonctions d'entrée artérielles locales pour chaque voxel considéré : en effet, si les signaux d'entrée artérielles ne sont pas adéquats et ne correspondent pas aux véritables fonctions d'entrée artérielles locales, il se peut qu'il n'y ait pas de jeu de paramètres (paramètres hémodynamiques, fonction de répartition complémentaire, etc.) qui soit solution du problème tout en respectant les différentes contraintes posées a priori (ou du moins dont la probabilité n'est pas négligeable a priori). Dans ce cas, la Théorie des Probabilités interprétera les signaux d'entrée artériels proposés comme du bruit : les écarts-types $\sigma_a/\varepsilon_a$ seront beaucoup plus grands par rapport à ceux typiquement obtenues à partir de signaux d'entrée artériels plus adéquats.

L'invention trouve ainsi une application particulièrement intéressante pour tester les différentes méthodes de sélection ou d'estimation de fonctions d'entrée artérielles globale ou locales suivant l'état de l'art. S'il s'avère que les estimations, en particulier celles sur leurs paramètres dé régularisation $E_a$, obtenues à partir de ces fonctions d'entrée artérielles globale ou locales sont trop souvent aberrantes d'un voxel à un autre, on pourra conclure qu'il faut soit introduire de nouvelles méthodes (locales) de sélection plus appropriées pour ces fonctions soit recourir à des méthodes ne nécessitant pas la donnée de fonctions d'entrée artérielles ou la mesure préalables de signaux d'entrée artériels, ce qui est précisément l'objet de la troisième méthode décrite précédemment.

A titre d'exemple d'application, nous pouvons citer les principales étapes de mise en oeuvre de l'invention au moyen d'un système d'analyse d'imagerie de perfusion adapté, tel que celui décrit en figures 1 ou 2 :

- ouverture d'un dossier patient ou prise en compte de séquences d'images par l'unité de traitement 4 (ou de prétraitement 7) pour sélectionner des séquences d'images d'intérêt - en particulier, sélection des images I1 à In de perfusion au cours du temps à partir desquelles sont obtenus les signaux de perfusion S($t$) pour chaque voxel, tel qu'illustré en figure 5a ;
- prévisualisation au moyen d'une interface homme-machine 5 des images pour permettre à un utilisateur 6 d'identifier des tranches ou des zones d'intérêt ;
- configuration de l'unité de traitement 4 à partir des paramètres de configuration (informations introduite) pour permettre la mise en oeuvre du procédé d'estimation conforme à l'invention ;
- choix de la (ou les) quantité(s) d'intérêt à estimer ;

- estimation par l'unité de traitement 4 de paramètres hémodynamiques 14, tels que $\widehat{BF}$, $\hat{\tau}$ ou $\widehat{MTT}$, pour un organe tel que le cerveau humain ;
- estimation optionnelle d'autres paramètres tels que $E_h$, $E_S$, $\Theta_S$ ou encore $E_a$, $E_{S_a}$ ou $\Theta_{S_a}$ lorsque la fonction d'entrée artérielle n'est pas donnée ;
- estimation optionnelle des vecteurs des valeurs des réponses impulsionnelles $\hat{h}$, des fonctions de répartition complémentaires $\hat{b}$, des vecteurs des valeurs des fonctions d'entrée artérielles $\hat{a}$, des vecteurs des valeurs des fonctions de sortie veineuses $\hat{v}$, des vecteurs des valeurs des concentrations théoriques $\hat{c}$, des signaux théoriques $\widehat{s_{th}}$ ou encore des résidus $\hat{r}$;
- délivrance desdites quantités d'intérêt estimées 14 à l'interface homme-machine 5 pour que celle-ci les présente *in fine* par exemple sous la forme de cartes où l'intensité ou la couleur de chaque pixel dépend de la valeur calculée pour en restituer la teneur au praticien ;
- affichage optionnel des estimations des réponses impulsionnelles, des fonctions de répartition complémentaires, des fonctions d'entrée artérielles, des fonctions de sortie veineuses, des concentrations théoriques, des signaux théoriques ou encore des résidus pour certains voxels d'intérêt choisis par l'utilisateur ;
- affichage optionnel de cartes de confiance ou de cartes de paris pour un ou plusieurs paramètres d'intérêt tels que les paramètres hémodynamiques ;
- affichage optionnel d'intervalles de confiance ou de paris sur ces intervalles de confiance pour certaines réponses impulsionnelles, fonctions de répartition complémentaires, fonctions d'entrée artérielles, etc. pour certains voxels d'intérêt choisis par l'utilisateur ;
- affichage optionnel de cartes d'erreur pour une ou plusieurs distances entre données expérimentales et modèle de perfusion non-paramétrique global, en particulier affichage de la probabilité des données expérimentales sachant le modèle de perfusion global ;
- sélection assistée de ladite zone pathologique d'intérêt par le praticien, caractérisée par une anomalie de la distribution d'un ou de plusieurs paramètres hémodynamiques, des réponses impulsionnelles (ou des fonctions de répartition complémentaires) ou des fonctions d'entrée artérielles locales;
- estimation, par l'unité de traitement, du volume de la zone de tissus anormalement perfusée éventuellement connexe à une zone lésée et pour laquelle le praticien pourra décider une action thérapeutique (thrombolyse intraveineuse afin de résorber le caillot sanguin par exemple) ;
- estimation, par l'unité de traitement, de certaines quantités, telles que le ratio des volumes des zones lésée et anormalement perfusée sur lesquelles, un praticien pourra peaufiner son diagnostic et sa prise de décision thérapeutique (thrombolyse intraveineuse afin de résorber un caillot sanguin par exemple).

Comme décrit précédemment, le processus de configuration 50 de l'unité de traitement 4 peut être réalisé par l'unité elle-même (mise en oeuvre du processus 50). En variante, ladite configuration peut consister à mémoriser et sélectionner une bibliothèque de distributions de probabilité conjointes a posteriori selon les quantités d'intérêt que l'on souhaite estimer. L'élaboration de cette bibliothèque peut être réalisée à l'aide d'une unité dédiée et apte à coopérer avec l'unité de traitement 4.

En variante, il peut exister des itérations à la suite de l'estimation d'intervalles de confiance, de paris sur ces intervalles de confiance pour certaines quantités d'intérêts afin d'affiner ladite configuration. L'élaboration de distances entre données expérimentales et modèle de perfusion non-paramétrique global, en particulier affichage de la probabilité des données expérimentales sachant le modèle de perfusion global peut également induire une mise à jour de la configuration.

L'invention prévoit donc d'afficher des estimations des paramètres sous forme de « cartes de paramètres » où l'intensité ou la couleur de chaque voxel dépend de la valeur calculée, par exemple de manière linéaire. De même, l'invention prévoit éventuellement d'afficher les écarts-types de ces estimations sous la forme de « cartes de confiance » ainsi que les paris sur les intervalles de confiance correspondant sous la forme de « cartes de paris ». Pour ce qui est des estimations des vecteurs de valeurs des réponses impulsionnelles, des fonctions de répartition complémentaires, des fonctions d'entrée artérielles, des fonctions de sortie veineuses, des courbes de concentration, des signaux de perfusion ou encore des résidus, l'invention prévoit leur affichage sous forme de séries temporelles pour chaque voxel où l'utilisateur le demande. Enfin, l'invention prévoit l'affichage de distances entre signaux expérimentaux et modèle de perfusion non-paramétrique ou de la probabilité des données expérimentales sachant ce modèle sous la forme de « cartes d'erreur ».

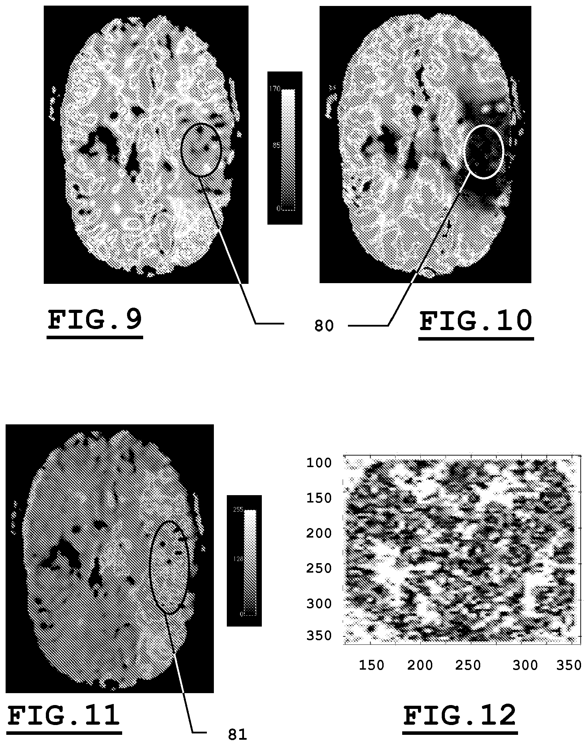

Les figures 9 à 12 permettent d'illustrer un mode d'affichage sous la forme de cartes, de certaines quantités d'intérêts tels que des paramètres hémodynamiques 14 estimés conformément à l'invention voire des écarts-types ou probabilités qui leur sont associés.

Ainsi pour un cerveau humain analysé à l'aide d'imagerie par Résonance Magnétique Nucléaire, la figure 9 permet de visualiser une estimation des volumes sanguins cérébraux (*cerebral blood volumes, CBV* suivant une terminologie anglo-saxonne). Une telle carte (458 x 458 pixels) permet de mettre en évidence une zone ischémique probable 80. En effet, il est possible de constater à l'aide d'une interface 6 adaptée, une nette augmentation du paramètre *CBV* dans le territoire de l'artère cérébrale postérieure droite par rapport à l'hémisphère controlatérale. Une vasodilatation consécutive à l'ischémie peut être révélée par une lecture de la carte telle qu'illustrée en figure 9.

La figure 10 permet d'illustrer une carte (458 x 458 pixels) relative à l'estimation des flux sanguins cérébraux en cas d'ischémie cérébrale. On peut constater en analysant la carte, une diminution du paramètre (*cerebral blood volumes, CBF* suivant une terminologie anglo-saxonne) dans le territoire de l'artère cérébrale postérieure droite par rapport à l'hémisphère controlatérale consécutive à l'ischémie. Une telle carte permet de mettre en évidence une zone ischémique probable 80.

La figure 11 permet d'illustrer une carte (458 x 458 pixels) relative à l'estimation des temps de transit moyen *MTT.* On peut constater en analysant la carte, une nette augmentation des *MTT* dans le territoire 81 de l'artère cérébrale postérieure droite par rapport à l'hémisphère controlatérale consécutive à l'ischémie.

La figure 12 permet de décrire une carte (458 x 458 pixels) relative à l'estimation de la probabilité que le flux sanguin cérébral soit dans l'intervalle de confiance $\left[ \widehat{CBF} - \hat{\sigma}_{CBF}, \widehat{CBF} + \hat{\sigma}_{CBF} \right]$. On peut constater en analysant la carte que, mis à part des ajustements aberrants, les probabilités sont centrées autour de 0,68. C'est une valeur à laquelle on est en droit de s'attendre si la distribution de probabilité a posteriori de *CBF* suit une loi normale.

Grâce aux cartes présentées précédemment, l'invention permet de mettre à la disposition d'un utilisateur tout un ensemble d'informations utiles, informations qui ne pouvaient être disponibles à l'aide des techniques connues de l'état de l'art. Cette mise à disposition est rendue possible par une adaptation de l'unité de traitement 4 selon les figures 1 ou 2 en ce que ses moyens pour communiquer avec le monde extérieur de l'unité 4 sont aptes à délivrer les paramètres estimés 14 selon un format approprié à une interface homme-machine 5 apte à restituer à un utilisateur 6 lesdits paramètres estimés sous la forme par exemple de cartes telles qu'illustrées par les figures 9 à 13.

Grâce à l'invention, les informations délivrées sont ainsi plus nombreuses et justes. Les informations dont dispose le praticien sont ainsi de nature à accroitre la confiance du praticien dans sa détermination d'un diagnostic et sa prise de décision thérapeutique.

Pour améliorer les performances du système selon l'invention, celle-ci prévoit que l'unité de traitement puisse être dotée de moyens pour paralléliser des calculs sur les voxels de l'image pour lesquels l'estimation des paramètres hémodynamiques, des fonctions de répartition complémentaires ou des fonctions d'entrée artérielles est requise. Cela peut être effectué en utilisant des technologies matérielles telles que les microprocesseurs graphiques (*Graphical Processor Unit* ou GPU, selon une terminologie anglo-saxonne) ou les grappes de calcul (clusters) ou logicielles telles que les Méthode de Monte-Carlo parallèles, etc. En variante, l'unité de traitement conforme à l'invention peut s'appuyer sur des moyens de calcul distants. Les temps de calculs peuvent ainsi être encore considérablement réduits.

## Revendications

**1.** Procédé pour estimer (57) une quantité d'intérêt (14) parmi une pluralité d'un système dynamique artère/tissu/veine d'un volume élémentaire - dit voxel - d'un organe, ledit procédé étant mis en oeuvre par une unité de traitement (4) d'un système d'analyse d'imagerie de perfusion, et comportant une étape pour estimer ladite quantité d'intérêt à partir d'une donnée (15) de perfusion expérimentale, ladite étape consistant à évaluer - suivant une méthode de Bayes - une distribution marginale a posteriori (56) pour ladite quantité d'intérêt par :

- l'assignation (54) d'une distribution de probabilité directe de la donnée de perfusion sachant les paramètres intervenant dans l'estimation des quantités d'intérêt du système dynamique artère/tissu/veine du voxel considéré ;
- l'assignation (53) d'une distribution de probabilité conjointe *a priori* desdites quantités, par l'introduction (51b) d'une information strictement douce sur la réponse impulsionnelle dudit système dynamique et par l'application du Principe du Maximum d'Entropie sur ladite réponse impulsionnelle dudit système dynamique afin d'obtenir la distribution de probabilité *a priori* de ladite réponse impulsionnelle dudit système dynamique.

2. Procédé pour estimer (57) une quantité d'intérêt (14) parmi une pluralité d'un système dynamique artère/tissu/veine d'un volume élémentaire - dit voxel - d'un organe, ledit système dynamique étant linéaire, invariant dans le temps et déterminé formellement par la relation $C(t)=BF \cdot C_a(t) \otimes R(t)$ où $C(t)$ est la concentration d'un agent de contraste circulant dans un voxel, $C_a(t)$ est la concentration dudit agent de contraste dans l'artère alimentant ledit voxel, $BF$ est le flux sanguin dans ledit voxel, $\otimes$ désigne le produit de convolution et $R(t)$ est la fonction de répartition complémentaire du temps de transit dans ledit voxel, ledit procédé étant mis en oeuvre par une unité de traitement (4) d'un système d'analyse d'imagerie de perfusion, et comportant une étape pour estimer ladite quantité d'intérêt à partir d'une donnée (15) de perfusion expérimentale, ladite étape consistant à évaluer (56), suivant une méthode de Bayes, une distribution marginale a posteriori pour ladite quantité d'intérêt par :

- l'assignation (54) d'une distribution de probabilité directe de la donnée de perfusion sachant les paramètres intervenant dans l'estimation des quantités d'intérêt du système dynamique artère/tissu/veine du voxel considéré ;
- l'assignation (53) d'une distribution de probabilité conjointe *a priori* desdites quantités, par l'introduction (51b) d'une information strictement douce sur la fonction de répartition complémentaire du temps de transit $R(t)$ dans ledit voxel et par l'application du Principe du Maximum d'Entropie sur R(t) afin d'obtenir la distribution de probabilité a priori de R(t).

3. Procédé selon l'une quelconque des revendications précédentes, **caractérisé en ce que** l'assignation (53) d'une distribution de probabilité conjointe *a priori* desdites quantités est réalisée en outre par l'introduction (51c) d'une information strictement douce sur la courbe de concentration dudit agent de contraste dans l'artère alimentant le voxel et par l'application du Principe du Maximum d'Entropie sur ladite courbe de concentration dudit agent de contraste afin d'obtenir la distribution de probabilité *a priori* de ladite courbe de concentration dudit agent de contraste.

4. Procédé selon l'une quelconque des revendications précédentes, **caractérisé en ce qu'**il est mis en oeuvre par itérations successives pour une pluralité de voxels considérés.

5. Procédé selon l'une quelconque des revendications précédentes, **caractérisé en ce qu'**il comporte une étape pour calculer (58) une information complémentaire sous la forme d'un intervalle de confiance associé à une quantité d'intérêt estimée.

6. Procédé selon l'une quelconque des revendications précédentes, **caractérisé en ce qu'**il comporte une étape pour calculer (59) une information complémentaire sous la forme d'un taux de pari sur un intervalle de confiance associé à une quantité d'intérêt estimée.

7. Procédé selon la revendication 4, **caractérisé en ce qu'**il comporte une étape pour calculer (60) une information complémentaire sous la forme d'une mesure de l'adéquation du produit de :

- l'assignation (54) de la distribution de probabilité directe de la donnée de perfusion sachant les paramètres intervenant dans le problème de l'estimation des quantités d'intérêt du système dynamique artère/tissu/veine du voxel considéré ;
- l'assignation (53) de la distribution de probabilité conjointe a priori desdites quantités.

8. Procédé selon l'une quelconque des revendications 4 à 7, **caractérisé en ce qu'**il comporte une étape pour délivrer une quantité d'intérêt estimée (14) à une interface homme-machine (5) apte à la restituer à un utilisateur (6).

9. Procédé selon l'une quelconque des revendications 5 à 8, **caractérisé en ce qu'**il comporte une étape pour délivrer toute information complémentaire associée à ladite quantité d'intérêt estimée, à une interface homme-machine (5) apte à la restituer à un utilisateur (6).

10. Procédé selon l'une quelconque des revendications précédentes, **caractérisé en ce que** la donnée de perfusion expérimentale consiste en un vecteur de valeurs d'un signal de perfusion expérimental ou en la conversion de ce dernier en un vecteur de valeurs d'une courbe de concentration.

11. Unité de traitement (4) comportant des moyens de mémorisation, des moyens pour communiquer avec le monde extérieur et des moyens de traitement, **caractérisée en ce que** :

- les moyens pour communiquer sont aptes à recevoir du monde extérieur une donnée de perfusion expérimentale (15);
- les moyens de traitement sont adaptés pour mettre en oeuvre un procédé pour estimer une quantité d'intérêt (14) parmi une pluralité d'un système dynamique artère/tissu/veine d'un volume élémentaire - dit voxel - d'un organe, selon l'une quelconques des revendications 1 à 10.

12. Unité de traitement selon la revendication précédente, **caractérisé en ce que** les moyens pour communiquer délivrent (57) une quantité d'intérêt estimée (14) selon un format approprié à une interface homme-machine (5) apte à la restituer à un utilisateur (6).

13. Unité de traitement selon la revendication précédente, caractérisé en que les moyens pour communiquer délivrent (58, 59) une information complémentaire associée à une quantité d'intérêt estimée (14) selon un format approprié à une interface homme-machine (5) apte à la restituer à un utilisateur (6).

14. Système d'analyse d'imagerie de perfusion comportant une unité de traitement (4) selon l'une quelconques des revendications 11 à 13 et une interface homme-machine (5) apte à restituer à un utilisateur (6) une quantité estimée (14) selon un procédé conforme à l'une quelconque des revendications 1 à 10 et mis en oeuvre par ladite unité de traitement (4).

**Patentansprüche**

1. Verfahren zum Schätzen (57) einer interessierenden Menge (14) in einer Vielzahl eines dynamischen Arterien-/Gewebe-/Venen-Systems mit einem elementaren Volumen - sogenanntes Voxel- eines Organs, wobei dieses Verfahren durch eine Verarbeitungseinheit (4) eines Analysesystems der Perfusionsbildgebung ausgeführt wird und einen Schritt zum Schätzen dieser interessierenden Menge auf Grundlage von Daten (15) einer experimentellen Perfusion umfasst, wobei der Schritt daraus besteht, - gemäß einem Bayes-Verfahren - *a posteriori* eine Randverteilung (56) für die interessierende Menge abzuschätzen, durch:

- Zuweisung (54) einer direkten Wahrscheinlichkeitsverteilung zu den Perfusionsdaten in Kenntnis der in die Schätzung der interessierenden Mengen des dynamischen Arterien-/Gewebe-/Venen-Systems des betrachteten Voxels in Betracht kommenden Parameter;
- Zuweisung (53) einer gemeinsamen *a priori* Wahrscheinlichkeitsverteilung dieser Mengen, durch die unbedingt langsame Einführung (51b) einer Information über die Impulsantwort des dynamischen Systems und durch die Anwendung des Prinzips der maximalen Entropie auf diese Impulsantwort dieses dynamischen Systems, um die *a priori* Wahrscheinlichkeitsverteilung dieser Impulsantwort des dynamischen Systems zu erhalten.

2. Verfahren zum Schätzen (57) einer interessierenden Menge (14) in einer Vielzahl eines dynamischen Arterien-/Gewebe-/Venen-Systems mit einem elementaren Volumen - sogenanntes Voxe - eines Organs, wobei das dynamische System linear ist, über die Zeit unveränderlich ist und formell durch die Beziehung C(t)=BF.C$_a$(t)⊗R(t) bestimmt wird, worin C(t) die Konzentration eines Kontrastmittels, das in einem Voxel zirkuliert, ist, C$_a$(t) die Konzentration dieses Kontrastmittels in der Arterie, welche das Voxel versorgt, ist, BF der Blutfluss in diesem Voxel ist, ⊗ das Produkt der Faltung bezeichnet und R(t) die Funktion der komplementären Aufteilung der Transitzeit in diesem Voxel ist, wobei das Verfahren durch eine Verarbeitungseinheit (4) eines Analysesystems einer bildgebenden Darstellung der Perfusion ausgeführt wird, und einen Schritt zum Schätzen dieser interessierenden Menge auf Grundlage von Daten (15) der experimentellen Perfusion zu schätzen, wobei der Schritt daraus besteht, gemäß einem Bayes-Verfahren eine a *posteriori* Randverteilung (56) für die interessierende Menge abzuschätzen, durch:

- Zuweisung (54) einer direkten Wahrscheinlichkeitsverteilung der Perfusionsdaten in Kenntnis der in die Schätzung der interessierenden Mengen des dynamischen Arterien-/Gewebe-/Venen-Systems des betrachteten Voxels in Betracht kommenden Parameter;
- Zuweisung (53) einer gemeinsamen *a priori* Wahrscheinlichkeitsverteilung der Mengen, durch die unbedingt langsame Einführung (51b) einer Information über die komplementäre Verteilungsfunktion der Transitzeit R(t) in dem Voxel und durch die Anwendung des Prinzips der maximalen Entropie auf R(t) um die *a priori* Wahrscheinlichkeitsverteilung von R(t) zu erhalten.

**3.** Verfahren nach einem der vorangehenden Ansprüche, **dadurch gekennzeichnet, dass** die Zuweisung (53) einer gemeinsamen a *priori* Wahrscheinlichkeitsverteilung der Mengen darüber hinaus durch die unbedingt langsame Einführung (51c) einer Information über die Konzentrationskurve des Kontrastmittels in der Arterie, welche das Voxel versorgt, erfolgt und durch die Anwendung des Prinzips der maximalen Entropie auf die Konzentrationskurve des Kontrastmittels erfolgt, um die *a priori* Wahrscheinlichkeitsverteilung der Konzentrationskurve des Kontrastmittels zu erhalten.

**4.** Verfahren nach einem der vorangehenden Ansprüche, **dadurch gekennzeichnet, dass** es durch aufeinanderfolgende Wiederholungen für eine Vielzahl der betrachteten Voxel durchgeführt wird.

**5.** Verfahren nach einem der vorangehenden Ansprüche, **dadurch gekennzeichnet, dass** es einen Schritt zur Berechnung (58) einer komplementären Information als ein Vertrauensintervall in Verbindung mit einer interessierenden geschätzten Menge enthält.

**6.** Verfahren nach einem der vorangehenden Ansprüche, **dadurch gekennzeichnet, dass** es einen Schritt zur Berechnung (59) einer komplementären Information als eine Wettquote auf ein Vertrauensintervall in Verbindung mit einer interessierenden geschätzten Menge enthält.

**7.** Verfahren nach Anspruch 4, **dadurch gekennzeichnet, dass** es einen Schritt zur Berechnung (60) einer komplementären Information in Form einer Messung der Übereinstimmung des Produkts durch:

- Zuweisung (54) einer direkten Wahrscheinlichkeitsverteilung zu den Perfusionsdaten in Kenntnis der in die Schätzung der interessierenden Mengen des dynamischen Arterien-/Gewebe-/Venen-Systems des betrachteten Voxels in Betracht kommenden Parameter;
- Zuweisung (53) der gemeinsamen *a priori* Wahrscheinlichkeitsverteilung der Mengen.

**8.** Verfahren nach einem der Ansprüche 4 bis 7, **dadurch gekennzeichnet, dass** es einen Schritt der Ausgabe einer interessierenden geschätzten Menge (14) an eine Schnittstelle Mensch-Maschine (5) umfasst, die dafür geeignet ist, diese an den Nutzer (6) zurückzugeben.

**9.** Verfahren nach einem der vorangehenden Ansprüche 5 bis 8, **dadurch gekennzeichnet, dass** es einen Schritt zur Ausgabe jeder mit der interessierenden geschätzten Menge verbundenen komplementären Information an eine Schnittstelle Mensch-Maschine (5) umfasst, die dafür geeignet ist, diese an einen Nutzer (6) zurückzugeben.

**10.** Verfahren nach einem der vorangehenden Ansprüche, dadurch charakterisiert, dass die experimentellen Perfusionsdaten in einem Vektor, welcher die Werte eines experimentellen Perfusionssignals umfasst oder in der Umwandlung dieses Vektors in einen Vektor, welcher die Werte einer Konzentrationskurve umfasst, bestehen.

**11.** Verarbeitungseinheit (4), welche Speichermittel, Mittel zur Kommunikation mit der Außenwelt und den Behandlungsmitteln umfasst, **dadurch gekennzeichnet, dass**:

- die Kommunikationsmittel für den Empfang von experimentellen Perfusionsdaten (15) von der Außenwelt geeignet sind;
- die Behandlungsmittel für die Durchführung eines Verfahrens zum Schätzen einer interessierenden Menge (14) in einer Vielzahl von dynamischen Arterien-/Gewebe-/Venen-Systemen mit elementarem Volumen - sogenanntes Voxel - eines Organs gemäß einem der Ansprüche 1 bis 10 angepasst sind.

**12.** Verarbeitungseinheit nach dem vorangehenden Anspruch, **dadurch gekennzeichnet, dass** die Kommunikationsmittel (57) eine interessierende geschätzte Menge (14) in einem Format ausgeben, das passend für eine Schnittstelle Mensch-Maschine (5) ist, die dafür geeignet ist, diese an den Nutzer (6) zurückzugeben.

**13.** Verarbeitungseinheit gemäß dem vorangehenden Anspruch, **dadurch gekennzeichnet, dass** die Kommunikationsmittel eine komplementäre Information ausgeben (58, 59), die mit einer interessierenden geschätzten Menge (14) in einem Format verbunden ist, das passend für eine Schnittstelle Mensch-Maschine (5) ist, die dafür geeignet ist, diese an den Nutzer (6) zurückzugeben.

**14.** Analysesystem der Perfusionsbildgebung, welches eine Verarbeitungseinheit (4) gemäß einem der Ansprüche 11 bis 13 und eine Schnittstelle Mensch-Maschine (5) umfasst, die dafür geeignet ist, eine geschätzte Menge (14) gemäß einem Verfahren gemäß den Ansprüchen 1 bis 10 an den Nutzer (6) zurückzugeben, wobei das Verfahren durch die Verarbeitungseinheit (4) ausgeführt wird.

**Claims**

**1.** Method for estimating (57) a quantity of interest (14) among a plurality of a dynamic artery/tissue/vein system of an elementary volume - referred to as a voxel - of an organ, said method being carried out by a processing unit (4) of a perfusion imaging analysis system, and comprising a step for estimating said quantity of interest from an experimental perfusion datum (15), said step consisting in evaluating - according to a Bayesian method - an *a posteriori* marginal distribution (56) for said quantity of interest by:

- the assignment (54) of a direct probability distribution of the perfusion datum given the parameters involved in the estimation of the quantities of interest of the dynamic artery/tissue/vein system of the voxel concerned;
- the assignment (53) of an *a priori* joint probability distribution of said quantities, by the introduction (51b) of a strictly soft information on the impulse response of said dynamic system and by the application of the Principle of Maximum Entropy on said impulse response of said dynamic system in order to obtain the *a priori* probability distribution of said impulse response of said dynamic system.

**2.** Method for estimating (57) a quantity of interest (14) among a plurality of a dynamic artery/tissue/vein system of an elementary volume - referred to as a voxel - of an organ, said dynamic system being linear, time invariant and formally determined by the relation $C(t)=BF.C_a(t) \otimes R(t)$ where $C(t)$ is the concentration of a contrast agent circulating in a voxel, $C_a(t)$ is the concentration of said contrast agent in the artery supplying said voxel, $BF$ is the blood flow in said voxel, $\otimes$ indicates the convolution product and $R(t)$ is the complementary cumulative density function of the transit time in said voxel, said method being carried out by a processing unit (4) of a perfusion imaging analysis system, and comprising a step to estimate said quantity of interest on the basis of an experimental perfusion datum (15) said step consisting in evaluating (56) - according to a Bayesian method - an *a posteriori* marginal distribution for said quantity of interest by:

- the assignment (54) of a direct probability distribution of the perfusion datum given the parameters involved in the estimation of the quantities of interest of the dynamic artery/tissue/vein system of the voxel concerned;
- the assignment (53) of an *a priori* joint probability distribution of said quantities, by the introduction (51b) of a strictly soft information on the cumulative density function of the transit time $R(t)$ in said voxel and by the application of the Principle of Maximum Entropy on $R(t)$ in order to obtain the *a priori* probability distribution of $R(t)$.

**3.** Method according to any of the preceding claims, **characterized in that** the assignment (53) of an *a priori* joint probability distribution of said quantities is also achieved by the introduction (51c) of a strictly soft information on the concentration curve of said contrast agent in the artery supplying the voxel and by the application of the Principle of Maximum Entropy on said concentration curve of said contrast agent in order to obtain the *a priori* probability distribution of said concentration curve of said contrast agent.

**4.** Method according to any of the preceding claims, **characterized in that** it is implemented by successive iterations for a plurality of voxels concerned.

**5.** Method according to any of the preceding claims, **characterized in that** it comprises a step for calculating (58) a supplementary information in the form of a confidence interval associated with an estimated quantity of interest.

**6.** Method according to any of the preceding claims, **characterized in that** it comprises a step for calculating (59) a supplementary information in the form of a bet rate on a confidence interval associated with an estimated quantity of interest.

**7.** Method according to claim 4, **characterized in that** it comprises a step for calculating (60) a supplementary information in the form of a measurement of the adequacy of the product of:

- the assignment (54) of the direct probability distribution of the perfusion datum given the parameters involved in the estimation of the quantities of interest of the dynamic artery/tissue/vein system of the voxel concerned;
- the assignment (53) of the *a priori* joint probability distribution of said quantities.

**8.** Method according to any of claims 4 to 7, **characterized in that** it comprises a step for delivering an estimated quantity of interest (14) to a man/machine interface (5) capable of rendering it to a user (6).

**9.** Method according to any of claims 5 to 8, **characterized in that** it comprises a step to deliver any supplementary information associated with said estimated quantity of interest, to a man/machine interface (5) capable of rendering it to a user (6).

**10.** Method according to any of the preceding claims, **characterized in that** the experimental perfusion datum consists in a vector of values of an experimental perfusion signal or in the conversion of the latter into a vector of values of a concentration curve.

**11.** Processing unit (4) comprising storage means, means to communicate with the outside world and processing means, **characterized in that**:

- the means to communicate are capable of receiving from the outside world an experimental perfusion datum (15);
- the processing means are configured to execute a method for estimating a quantity of interest (14) among a plurality of a dynamic artery/tissue/vein system of an elementary volume - referred to as a voxel - of an organ, according to any of claims 1 to 10.

**12.** Processing unit according to the preceding claim, **characterized in that** the means to communicate deliver (57) an estimated quantity of interest (14) in an appropriate format to a man/machine interface (5) capable of rendering it to a user (6).

**13.** Processing unit according to the preceding claim, **characterized in that** the communication means deliver (58, 59) supplementary information associated with an estimated quantity of interest (14) in an appropriate format to a man-machine interface (5) capable of rendering it to a user (6).

**14.** Perfusion imaging analysis system comprising a processing unit (4) according to any of claims 11 to 13 and a man/machine interface (5) capable of rendering to a user (6) an estimated quantity (14) using a method according to any of claims 1 to 10 and implemented by said processing unit (4).

FIG.1

FIG.2

FIG.3

FIG.4

**FIG.5a**

**FIG.5b**

**FIG.6**

**FIG.7**

**FIG.8**

FIG.9

FIG.10

FIG.11

FIG.12

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

• **SCHMID et al.** *IEEE Trans. Med. Imaging,* 2009, vol. 28, 789-798